# EUROPEAN PATENT APPLICATION

(11) **EP 4 226 949 A1**
(43) Date of publication of application: **16.08.2023**
(21) Application number: 22156604.5
(22) Date of filing: 14.02.2022
(51) Int. Cl.: A61K 48/00, A61K 9/00, A61K 9/51, C12N 15/00, A61K 9/127, A61K 31/7052

(54) **PREPARATION FOR USE IN A METHOD FOR THE TREATMENT AND/OR PREVENTION OF A DISEASE**

(71) Applicant: Pantherna Therapeutics GmbH, 16761 Hennigsdorf (DE)
(72) Inventor: The designation of the inventor has not yet been filed
(74) Representative: Bohmann, Armin K.

(57) **Abstract**

The present invention is related to a preparation for use in therapy,
wherein therapy comprises local administration of the preparation to a subject, wherein the local administration is selected from the group consisting of intramuscular administration, subcutaneous administration, intravitreal administration, intrathecal administration, intratumoral administration, intracerebral administration and intradermal administration,
wherein the preparation comprises lipid nanoparticles,
wherein the lipid nanoparticles comprise a lipid composition comprising a first and a second lipid, and a therapeutically active nucleic acid molecule, and,
wherein the lipid nanoparticles are amphoteric, overall neutrally charged lipid nanoparticles

## Description

The present invention is related to a preparation comprising a payload molecule and a lipid composition, the preparation for local delivery of the payload, the preparation for use in a method for the treatment and/or prevention of a disease, the preparation for use in a method for local delivery of the payload, a pharmaceutical composition comprising the preparation, the pharmaceutical composition for local delivery of the payload, the pharmaceutical composition for use in a method for the treatment and/or prevention of a disease, and a method for preparing said preparation.

Currently applied lipid nanoparticles (LNPs) for the intramuscular, subcutaneous, and intradermal administration of RNA are based on strongly cationic and even more often on pH-sensitive cationic lipids, also called ionizable cationic lipids.

The strongly cationic LNPs are made of a lipid system that contains beside neutral co-lipids and PEGylated lipids a strong cationic lipid. The pKa of said strong cationic lipid is usually above 9 which means that said cationic lipid is positively charged over a broad pH range. Such strong cationic lipid is thus positively charged at acidic (pH 3 to 6.7) and neutral pH values (pH 6.8 to 7.4) and do even show permanent cationic properties at mild (pH 7.5 to 8) and medium basic (pH 8.1 to 10) pH values. This characteristic allows a strong complexation of overall negatively charged ribonucleic acids (RNAs) and a strong interaction with overall negatively charged cell membrane, like endosomal membranes, that are intended to be transfected by LNPs comprising such strong cationic lipids. However, the strong cationic charges of this type of lipid interact with other negatively charged components such as extracellular substances like proteins, whereupon the complex comprising RNA and lipids rapidly dissociates, and the thus released RNA is rapidly degraded. Such interaction with negatively charged extracellular substances may also lead to aggregation of the lipid nanoparticles, hence inhibiting a functional uptake of the LNPs into the target cells.

On the other hand, pH-sensitive cationic LNPs are made of neutral co-lipids, PEGylated lipids and an ionizable cationic lipid that is characterized by a typical pKa value between 8 and 9.5. Due to the relatively low pKa value, such cationic lipid is positively charged at acidic pH values (typically at a pH of 4.5 to 5.5) and at these acidic pH values it is capable of interacting electrostatically with RNAs, or complexing RNAs and forming LNPs that encapsulate RNA, accordingly. Once the LNPs are formed and the pH of the suspension medium is adjusted to a neutral pH value, the pH-sensitive lipids at the surface of the LNPs are losing their positive charges, e.g., due to deprotonation of amino groups, whereas the RNAs are still entrapped inside the LNP particle. As a result, the LNP surface can be described as rather neutral and hydrophobic. These LNPs are originally designed as hepatocyte targeting LNPs for intravenous application and are known for their strong interaction with hydrophobic substances like ApoE.

The problem underlying the present invention is the provision of a means for delivering a payload, preferably a payload which is negatively charged at a neutral or physiological pH value, more preferably the payload is a nucleic acid molecule and most preferably an RNA molecule.

Another problem underlying the present invention is the provision of a means suitable for local administration of a payload, preferably a payload which is negatively charged at a neutral or physiological pH value, more preferably the payload is a nucleic acid molecule and most preferably an RNA molecule.

Another problem underlying the present invention is the provision of a means for local administration of a payload, preferably a payload which is negatively charged at a neutral or physiological pH value, more preferably the payload is a nucleic acid molecule and most preferably an RNA molecule.

Another problem underlying the present invention is the provision of a means suitable for local delivery of a payload, preferably a payload which is negatively charged at a neutral or physiological pH value, more preferably the payload is a nucleic acid molecule and most preferably an RNA molecule.

Another problem underlying the present invention is the provision of a means for local delivery of a payload, preferably a payload which is negatively charged at a neutral or physiological pH value, more preferably the payload is a nucleic acid molecule and most preferably an RNA molecule.

Another problem underlying the present invention is the provision of a means suitable for the treatment and/or prevention of a disease, wherein treatment and/or prevention of a disease comprises local administration of a payload, preferably a payload which is negatively charged at a neutral or physiological pH value, more preferably the payload is a nucleic acid molecule and most preferably an RNA molecule.

Another problem underlying the present invention is the provision of a means for the treatment and/or prevention of a disease, wherein treatment and/or prevention of a disease comprises local administration of a payload, preferably a payload which is negatively charged at a neutral or physiological pH value, more preferably the payload is a nucleic acid molecule and most preferably an RNA molecule.

A still further problem underlying the present invention is the provision of a method for producing such means.

These and other problems are solved by the subject matter of the attached independent claims; preferred embodiments may be taken from the attached dependent claims.

Furthermore, these and other problems are also solved by the subject matter of the following aspects and embodiments thereof.

The problem underlying the present invention is solved in a first aspect, which is also a first embodiment of the first aspect, by a preparation comprising a payload molecule and a lipid composition, wherein the lipid composition comprises a first lipid and a second lipid, wherein the first lipid is an anionic lipid having at least one molecular moiety with a pKa value between 4 and 7, and the second lipid is (a) a cationic lipid having at least one molecular moiety with a pKa value between greater 9.5 and 13.5, or (b) a cationic lipid which is pH-independently positively charged.

In a second embodiment of the first aspect which is also an embodiment of the first embodiment of the first aspect, the pKa value of the first lipid is between 5 and 7.

In a third embodiment of the first aspect which is also an embodiment of the first and the second embodiment of the first aspect, the pKa value of the first lipid is between 5.5 and 6.5.

In a fourth embodiment of the first aspect which is also an embodiment of the first, second and third embodiment of the first aspect, the pKa value of the at least one molecular moiety of the second lipid is 9.5 < pKa ≤ 13.5, preferably the pKa value is between 9.8 and 13.2.

In a fifth embodiment of the first aspect which is also an embodiment of the first, second, third and fourth embodiment of the first aspect, the pKa value of the at least one molecular moiety of the second lipid is between 10 and 13.

In a sixth embodiment of the first aspect which is also an embodiment of the first, second and third embodiment of the first aspect, the second lipid is a lipid which is pH-independently positively charged.

In a seventh embodiment of the first aspect which is also an embodiment of the first, second, third, fourth, fifth and sixth embodiment of the first aspect, the first lipid is bearing at least one negative charge.

In an eighth embodiment of the first aspect which is also an embodiment of the seventh embodiment of the first aspect, the first lipid is bearing at least one negative charge at a neutral pH value.

In a ninth embodiment of the first aspect which is also an embodiment of the eighth embodiment of the first aspect, the neutral pH value is a pH value of about 6.7 to about 7.7, preferably a pH value of about 6.9 to about 7.5, more preferably a pH value of about 7.2 to about 7.5.

In a tenth embodiment of the first aspect which is also an embodiment of any one of the first, second, third, fourth, fifth, sixth, seventh, eighth and ninth embodiment of the first aspect, the second lipid is bearing at least one positive charge.

In an eleventh embodiment of the first aspect which is also an embodiment of the tenth embodiment of the first aspect, the second lipid is bearing at least one positive charge at a neutral pH value.

In a twelfth embodiment of the first aspect which is also an embodiment of the eleventh embodiment of the first aspect, the neutral pH value is a pH value of about 6.7 to about 7.7, preferably a pH value of about 6.9 to about 7.5, more preferably a pH value of about 7.2 to about 7.5.

In a 13^{th} embodiment of the first aspect which is also an embodiment of any one of the first, second, third, fourth, fifth, sixth, seventh, eighth, ninth, tenth, eleventh and twelfth embodiment of the first aspect, the first lipid is bearing at least one carboxylic group.

In a 14^{th} embodiment of the first aspect which is also an embodiment of any one of the first, second, third, fourth, fifth, sixth, seventh, eighth, ninth, tenth, eleventh, twelfth and 13^{th} embodiment of the first aspect, preferably of the 13^{th} embodiment of the first aspect, the second lipid is bearing at least one group selected from the group comprising an amino group, an imino group, a guanidino group, an amino group substituted with an alkyl group, an imino group substituted with an alkyl group and a guanidino group substituted with an alkyl group.

In a 15^{th} embodiment of the first aspect which is also an embodiment of the 14^{th} embodiment of the first aspect, the alkyl group is a straight and/or branched chain C1, C2, C3 or C4 hydrocarbon group.

In a 16^{th} embodiment of the first aspect which is also an embodiment of any one of the first, second, third, fourth, fifth, sixth, seventh, eighth, ninth, tenth, eleventh, twelfth, 13^{th}, 14^{th} and 15^{th} embodiment of the first aspect, the lipid composition comprises a third lipid.

In a 17^{th} embodiment of the first aspect which is also an embodiment of the 16^{th} embodiment of the first aspect, the third lipid is a neutral lipid.

In an 18^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th} and 17^{th} embodiment of the first aspect, the lipid composition comprises a fourth lipid.

In a 19^{th} embodiment of the first aspect which is also an embodiment of the 18^{th} embodiment of the first aspect, the fourth lipid is a PEGylated lipid.

In a 20^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th} and 19^{th} embodiment of the first aspect, the lipid composition forms lipid nanoparticles.

In a 21^{st} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th} and 19^{th} embodiment of the first aspect, the lipid composition comprises lipid nanoparticles.

In a 22^{nd} embodiment of the first aspect which is also an embodiment of the 20^{th} embodiment and the 21^{st} embodiment of the first aspect, a surface of the lipid nanoparticles provides about a same number of positive charges and of negative charges.

In a 23^{rd} embodiment of the first aspect which is also an embodiment of the 22^{nd} embodiment of the first aspect, the surface of the lipid nanoparticles is an outer surface of the lipid nanoparticles.

In a 24^{th} embodiment of the first aspect which is also an embodiment of the 20^{th} and the 21^{st} embodiment of the first aspect, about a same number of positive charges and of negative charges is present on a surface of the lipid nanoparticles.

In a 25^{th} embodiment of the first aspect which is also an embodiment of the 24^{th} embodiment of the first aspect, the surface of the lipid nanoparticles is an outer surface of the lipid nanoparticles.

In a 26^{th} embodiment of the first aspect which is also an embodiment of the 22^{nd,} 23^{rd}, 24^{th} and 25^{th} embodiment of the first aspect, the number of positive charges and of negative charges is the number of positive (cationic) and negative (anionic) charges each at a neutral pH value.

In a 27^{th} embodiment of the first aspect which is also an embodiment of the 26^{th} embodiment of the first aspect, the neutral pH value is a pH value of about 6.7 to about 7.7, preferably of about 6.9 to about 7.5, more preferably of about 7.2 to about 7.5.

In a 28^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th} and 27^{th} embodiment of the first aspect, the lipid composition comprises the first and the second lipid in a dispersion medium.

In a 29^{th} embodiment of the first aspect which is also an embodiment of the 28^{th} embodiment of the first aspect, the dispersion medium comprises the lipid nanoparticles.

In a 30^{th} embodiment of the first aspect which is also an embodiment of the 28^{th} and the 29^{th} embodiment of the first aspect, the dispersion medium comprises a continuous phase.

In a 31^{st} embodiment of the first aspect which is also an embodiment of the 30^{th} embodiment of the first aspect, the continuous phase comprises a pharmaceutically acceptable aqueous buffer system, preferably the buffer system has a physiological pH value and isoosmolar strength and further comprises a cryoprotectant, more preferably the buffer system has a pH value of 7.0 to 7.4 and an osmolality of 250 to 330 mosmol/kg and comprises a cryoprotectant selected from a group comprising sucrose and trehalose, and even more preferably the buffer system is a 10 mM phosphate or 10 mM TRIS/HCl buffer of pH 7.4 and comprises 270 mM Sucrose as cryoprotectant.

In a 32^{nd} embodiment of the first aspect which is also an embodiment of the 28^{th}, 29^{th}, 30^{th} and 31^{st} embodiment of the first aspect, the pH value of the dispersion medium is a neutral pH value.

In a 33^{rd} embodiment of the first aspect which is also an embodiment of the 32^{nd} embodiment of the first aspect, the neutral pH value is a pH value of about 6.7 to about 7.7, preferably of about 6.9 to about 7.5, more preferably of about 7.2 to about 7.5.

In a 34^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd} embodiment of the first aspect, the molar ratio of the first lipid to the second lipid is such that the number of negative (anionic) charges provided by the lipid composition and the number of positive (cationic) charges provided by the lipid composition is about identical.

In a 35^{th} embodiment of the first aspect which is also an embodiment of the 34^{th} embodiment of the first aspect, the molar ratio of the first lipid of the lipid composition to the second lipid of the lipid composition is such that the number of negative charges provided by the lipid composition on a surface of the lipid nanoparticles is about the same as the number of positive charges provided by the lipid composition on a surface of the lipid nanoparticles.

In a 36^{th} embodiment of the first aspect which is also an embodiment of the 35^{th} embodiment of the first aspect, the surface of the lipid nanoparticles is an outer surface of the lipid nanoparticles.

In a 37^{th} embodiment of the first aspect which is also an embodiment of the 35^{th} and the 36^{th} embodiment of the first aspect, the number of negative charges provided by the lipid composition on a surface of the lipid nanoparticles is about the same as the number of positive charges provided by the lipid composition on a surface of the lipid nanoparticles at a neutral pH value.

In a 38^{th} embodiment of the first aspect which is also an embodiment of the 37^{th} embodiment of the first aspect, the neutral pH value is a pH value of about 6.7 to about 7.7, preferably of about 6.9 to about 7.5, more preferably of about 7.2 to about 7.5.

In a 39^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd} embodiment of the first aspect, the molar ratio of the first lipid to the second lipid is such that the number of negative (anionic) charges of the lipid composition and the number of positive (cationic) charges of the lipid composition is about identical.

In a 40^{th} embodiment of the first aspect which is also an embodiment of the 39^{th} embodiment of the first aspect, the molar ratio of the first lipid of the lipid composition to the second lipid of the lipid composition is such that the number of negative charges of the lipid composition on a surface of the lipid nanoparticles is about the same as the number of positive charges of the lipid composition on a surface of the lipid nanoparticles.

In a 41st embodiment of the first aspect which is also an embodiment of the 40^{th} embodiment of the first aspect, the surface of the lipid nanoparticles is an outer surface of the lipid nanoparticles.

In a 42^{nd} embodiment of the first aspect which is also an embodiment of the 40^{th} and the 41^{st} embodiment of the first aspect, the number of negative charges of the lipid composition on a surface of the lipid nanoparticles is about the same as the number of positive charges of the lipid composition on a surface of the lipid nanoparticles at a neutral pH value.

In a 43^{rd} embodiment of the first aspect which is also an embodiment of the 42^{nd} embodiment of the first aspect, the neutral pH value is a pH value of about 6.7 to about 7.7, preferably of about 6.9 to about 7.5, more preferably of about 7.2 to about 7.5.

In a 44^{th} embodiment of the first aspect which is also an embodiment of the 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd} and 43^{rd} embodiment of the first aspect, the lipid nanoparticles are amphoteric lipid nanoparticles.

In a 45^{th} embodiment of the first aspect which is also an embodiment of the 44^{th} embodiment of the first aspect, the lipid nanoparticles are amphoteric lipid nanoparticles at a pH value of about 7 to about 8, preferably at a pH of about 7.4

In a 46^{th} embodiment of the first aspect which is also an embodiment of the 44^{th} and the 45^{th} embodiment of the first aspect, the amphoteric lipid nanoparticles are overall neutrally charged lipid nanoparticles.

In a 47^{th} embodiment of the first aspect which is also an embodiment of the 46^{th} embodiment of the first aspect, the amphoteric lipid nanoparticles are overall neutrally charged lipid nanoparticles at a pH value of about 7 to about 8, preferably at a pH of about 7.4.

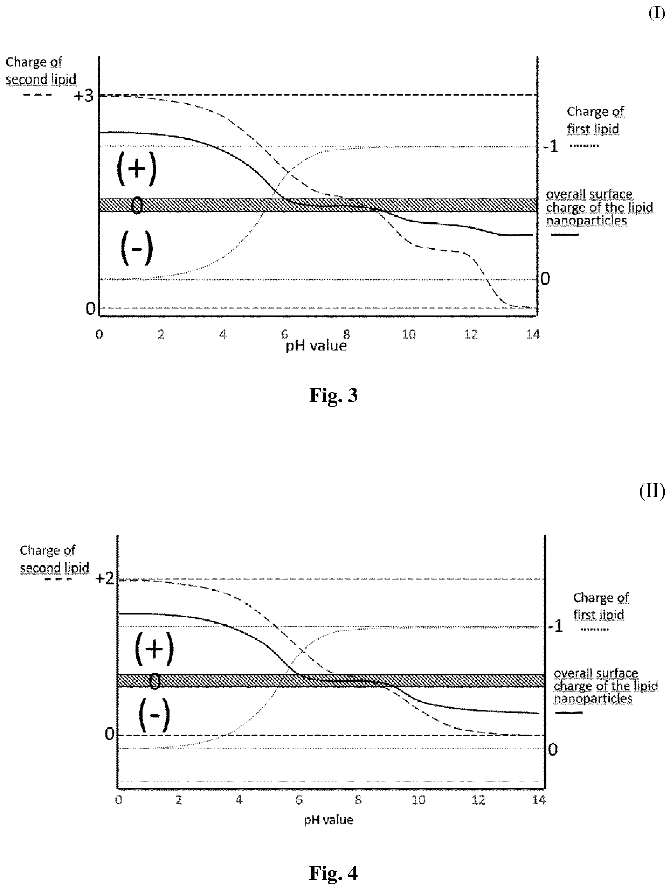

In a 48^{th} embodiment of the first aspect which is also an embodiment of the 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th} and 47^{th} embodiment of the first aspect, the first lipid is bearing a carboxylic group which is deprotonated in a pH-dependent manner, and the second lipid is bearing three functional groups which are protonated in a pH-dependent manner, wherein the lipid nanoparticles have a pH-dependent overall surface charge as subject to diagram (I) shown in Fig. 3.

In a 49^{th} embodiment of the first aspect which is also an embodiment of the 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th} and 47^{th} embodiment of the first aspect, the first lipid is bearing a carboxylic group which is deprotonated in a pH-dependent manner, and the second lipid is bearing two functional groups which are protonated in a pH-dependent manner, wherein the lipid nanoparticles have a pH-dependent overall surface charge as subject to diagram (II) shown in Fig. 4.

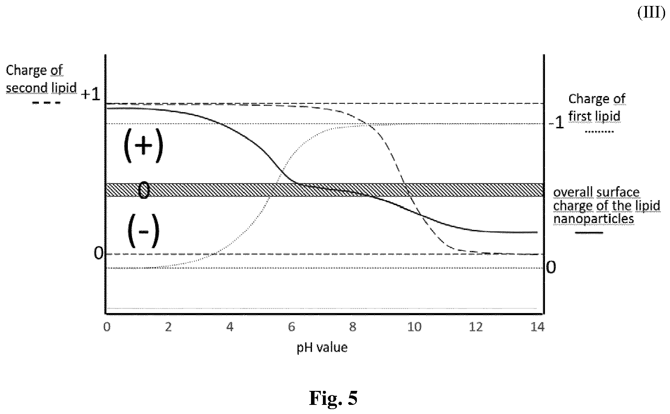

In a 50^{th} embodiment of the first aspect which is also an embodiment of the 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th} and 47^{th} embodiment of the first aspect, the first lipid is bearing a carboxylic group which is deprotonated in a PH-dependent manner, and the second lipid is bearing one functional group which is protonated in a pH-dependent manner, wherein the lipid nanoparticles have a pH-dependent overall surface charge as subject to diagram (III) shown in Fig. 5.

In a 51^{st} embodiment of the first aspect which is also an embodiment of the 48^{th}, 49^{th} and 50^{th} embodiment of the first aspect, the pH-dependent overall surface charge of the lipid nanoparticles results from the negative charges provided by the first lipid of the lipid composition and from the positive charges provided by the second lipid of the lipid composition.

In a 52^{nd} embodiment of the first aspect which is also an embodiment of the 48^{th}, 49^{th}, 50^{th} and 51^{st} embodiment of the first aspect, the overall surface charge of the lipid nanoparticles is the overall surface charge of the lipid particles present in the dispersion medium.

In a 53^{rd} embodiment of the first aspect which is also an embodiment of the 52^{nd} embodiment of the first aspect, the overall surface charge of the lipid nanoparticles is the overall charge of an outer surface of the lipid nanoparticles.

In a 54^{th} embodiment of the first aspect which is also an embodiment of the 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd} and 53^{rd} embodiment of the first aspect, the lipid nanoparticles are hydrophilic nanoparticles.

In a 55^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd} and 54^{th} embodiment of the first aspect, the first lipid is selected from the group comprising CHEMS (cholesterol hemisuccinate), alkyl carboxylic acids, diacyl glycerol hemisuccinates, and diacyl phosphatidylserines.

In a 56^{th} embodiment of the first aspect which is also an embodiment of the 55^{th} embodiment of the first aspect, the acyl group is a saturated or unsaturated branched or straight chain alkyl group of 12 to 20 hydrocarbons, i.e. 12, 13, 14, 15, 16, 17, 18, 19 or 20 hydrocarbons, preferably the acyl group is an unsaturated straight chain alkyl group of 12, 13, 14, 15, 16, 17, 18, 19 or 20 hydrocarbons.

In a 57^{th} embodiment of the first aspect which is also an embodiment of the 55^{th} and 56^{th} embodiment of the first aspect, the first lipid is CHEMS.

In a 58^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd} 53^{rd}, 54^{th}, 55^{th}, 56^{th} and 57^{th} embodiment of the first aspect, the second lipid is selected from the group comprising β-(L-arginyl)-L-2,3-diamino propionic acid-N-palmityl-N-oleyl-amide, L-arginyl-β-alanine-N-palmityl-N-oleyl-amide, DOTAP (N-[1-(2,3-dioleoyloxy)propyl]-N,N,N-trimethylammonium), DOTMA (1,2-di-O-octadecenyl-3-trimethylammonium propane), and DC-Cholesterol (3β-[N-(N',N'-dimethylaminoethane)-carbamoyl]cholesterol hydrochloride).

In a 59^{th} embodiment of the first aspect which is also an embodiment of the 58^{th} embodiment of the first aspect, DOTAP is (N-[1-(2,3-dioleoyloxy)propyl]-N,N,N-trimethylammonium chloride) or any other salt thereof such as a mesylate, although the chloride salt is preferred.

In a 60^{th} embodiment of the first aspect which is also an embodiment of the 58th embodiment of the first aspect, DOTMA is 1,2-di-O-octadecenyl-3-trimethylammonium propane as a chloride salt or any other salt thereof such as a mesylate, although the chloride salt is preferred.

In a 61^{st} embodiment of the first aspect which is also an embodiment of the 58^{th} embodiment of the first aspect, DC-Cholesterol is 3β-[N-(N',N'-dimethylaminoethane)-carbamoyl]cholesterol hydrochloride or any other salt thereof such as a mesylate, although the chloride salt is preferred.

In a 62^{nd} embodiment of the first aspect which is also an embodiment of the 58^{th} embodiment of the first aspect, the second lipid is selected from a group comprising β-(L-Arginyl)-L-2,3-diamino propionic acid-N-palmityl-N-oleyl-amide and L-arginyl-β-alanine-N-palmityl-N-oleyl-amide, preferably, the second lipid is β-(L-Arginyl)-L-2,3-diamino propionic acid-N-palmityl-N-oleyl-amide.

In a 63^{rd} embodiment of the first aspect which is also an embodiment of the 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st} and 62^{nd} embodiment of the first aspect, the third lipid is a zwitterionic phospholipid or an uncharged sterol lipid.

In a 64^{th} embodiment of the first aspect which is also an embodiment of the 63^{rd} embodiment of the first aspect, the zwitterionic phospholipid is selected from the group comprising phosphatidyl ethanolamine and phosphatidyl choline.

In a 65^{th} embodiment of the first aspect which is also an embodiment of the 63^{rd} embodiment of the first aspect, the uncharged sterol lipid is selected from the group comprising a zoosterol and a phytosterol.

In a 66^{th} embodiment of the first aspect which is also an embodiment of the 63^{rd} and the 64^{th} embodiment of the first aspect, the zwitterionic phospholipid is selected from the group comprising DSPC (1,2-Distearoyl-sn-glycero-3-phosphocholine), DMPC (1,2-dimyristoyl-sn-glycero-3-phosphocholine), DSPE (1,2-distearoyl-sn-glycero-3-phosphoethanolamine), DMPE (1,2-dimyristoyl-sn-glycero-3-phosphoethanolamine), DLPE (1,2-Lauroyl-sn-glycero-3 -phosphoethanolamine), DPhyPE (1,2-Diphytanoyl-sn-glycero-3 -phosphoethanolamine), DOPE (1,2-Dioleoyl-sn-glycero-3-phosphoethanolamine), and POPE (1-palmitoyl-2-oleoyl-sn-glycero-3 -phosphoethanolamine).

In a 67^{th} embodiment of the first aspect which is also an embodiment of the 63^{rd} embodiment of the first aspect, the uncharged sterol lipid is selected from the group comprising cholesterol and stigmasterol.

In a 68^{th} embodiment of the first aspect which is also an embodiment of the 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th} and 67^{th} embodiment of the first aspect, the fourth lipid is a shielding lipid.

In a 69^{th} embodiment of the first aspect which is also an embodiment of the 68^{th} embodiment of the first aspect, the shielding lipid is a lipid comprising a moiety selected from the group comprising PEG (polyethylene glycol), HES (hydroxy ethyl starch), PG (polyglycines) and polySarc (poly-sarcosine (poly-N-methylglycine)).

In a 70^{th} embodiment of the first aspect which is also an embodiment of the 69^{th} embodiment of the first aspect, the shielding lipid is a PEGylated lipid.

In a 71^{st} embodiment of the first aspect which is also an embodiment of the 70^{th} embodiment of the first aspect, the PEGylated lipid is selected from the group comprising methoxyPEG-DSPE, methoxyPEG-DMPE, methoxyPEG-DMG, methoxyPEG-DPG, methoxyPEG-DSG, methoxyPEG-c-DMA, 2(methoxyPEG)-N,N-dioctadecylacetamide, 2(methoxyPEG)-N,N-ditetradecylacetamide, methoxyPEG-C8-Ceramide, and methoxyPEG-C16-Ceramide.

In a 72^{nd} embodiment of the first aspect which is also an embodiment of any one of the 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, and 71^{st} embodiment of the first aspect, the PEG moiety of the PEGylated lipid has a molecular weight of from about 750 Da to about 5000 Da, preferably from about 1500 Da to about 3000 Da.

In a 73^{rd} embodiment of the first aspect which is also an embodiment of the 72^{nd} embodiment of the first aspect, the PEG is a straight PEG or a branched PEG.

In a 74^{th} embodiment of the first aspect which is also an embodiment of the 73^{rd} embodiment of the first aspect, the PEG is a straight PEG.

In a 75th embodiment of the first aspect which is also an embodiment of any one of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th} 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd} and 74^{th} embodiment of the first aspect, preferably of the 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd} and 74^{th} embodiment of the first aspect, the molar percentages of the lipids of the lipid composition is as follows:
- 20 - 80 mol% of the first lipid
- 20 - 80 mol% of the second lipid
- 10 - 50 mol% of the third lipid, and
- 1 - 10 mol% of the fourth lipid,
wherein the overall lipid content of the lipid composition is 100 mol%.

In a 76th embodiment of the first aspect which is also an embodiment of the 75th embodiment of the first aspect, the molar ratio of the lipid composition is as follows:
- 20 - 60 mol% of the first lipid
- 20 - 60 mol% of the second lipid
- 10 - 40 mol% of the third lipid, and
- 1 - 5 mol% of the fourth lipid,
wherein the overall lipid content of the lipid composition is 100 mol%.

In a 77th embodiment of the first aspect which is also an embodiment of any one of the first, second, third, fourth, fifth, sixth, seventh, eighth, ninth, tenth, eleventh, twelfth, 13th, 14th, 15th, 16th, 17th, 18th, 19th, 20th, 21st, 22nd, 23rd, 24th, 25th, 26th, 27th, 28th, 29th, 30th, 31st, 32nd, 33rd, 34th, 35th, 36th, 37th, 38th, 39th, 40th, 41st, 42nd, 43rd, 44th, 45th, 46th, 47th, 48th, 49th, 50th, 51st, 52nd, 53rd, 54th, 55th, 56th, 57th, 58th, 59th, 60th, 61st, 62nd, 63rd, 64th, 65th, 66th, 67th, 68th, 69th, 70th, 71st, 72nd, 73rd, 74th, 75th and 76th embodiment of the first aspect, preferably of the 18th, 19th, 20th, 21st, 22nd, 23rd, 24th, 25th, 26th, 27th, 28th, 29th, 30th, 31st, 32nd, 33rd, 34th, 35th, 36th, 37th, 38th, 39th, 40th, 41st, 42nd, 43rd, 44th, 45th, 46th, 47th, 48th, 49th, 50th, 51st, 52nd, 53rd, 54th, 55th, 56th, 57th, 58th, 59th, 60th, 61st, 62nd, 63rd, 64th, 65th, 66th, 67th, 68th, 69th, 70th, 71st, 72nd, 73rd, 74th, 75th and 76th embodiment of the first aspect, the lipid composition comprises:
- CHEMS as the first lipid,
- β-(L-Arginyl)-L-2,3-diamino propionic acid-N-palmityl-N-oleyl-amide as the second lipid,
- cholesterol as the third lipid,
- methoxyPEG2000-DMPE as the fourth lipid.

In a 78th embodiment of the first aspect which is also an embodiment of the77th embodiment of the first aspect, the lipid composition comprises
- 49 mol% of CHEMS as the first lipid,
- 29 mol% of β-(L-Arginyl)-L-2,3-diamino propionic acid-N-palmityl-N-oleyl-amide as the second lipid,
- 20 mol% of cholesterol as the third lipid and
- 2 mol% mPEG2000-DMPE as the fourth lipid.

In a 79^{th} embodiment of the first aspect which is also an embodiment of any one of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th} and 76^{th} embodiment of the first aspect, preferably of the 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th} , 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th} and 76^{th} embodiment of the first aspect, the lipid composition comprises:
- CHEMS as the first lipid
- β-(L-Arginyl)-L-2,3-diamino propionic acid-N-palmityl-N-oleyl-amide as the second lipid
- cholesterol as the third lipid and
- methoxyPEG2000-DMPE as the fourth lipid.

In an 80^{th} embodiment of the first aspect which is also an embodiment of the 79^{th} embodiment of the first aspect, the lipid composition comprises
- 48 mol% of CHEMS as the first lipid
- 29 mol% of β-(L-Arginyl)-L-2,3-diamino propionic acid-N-palmityl-N-oleyl-amide as the second lipid
- 18 mol% of cholesterol as the third lipid and
- 5 mol% of methoxyPEG2000-DMPE as the fourth lipid.

In a 81st embodiment of the first aspect which is also embodiment of any one of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th} and 76^{th} embodiment of the first aspect, the lipid composition is dissolved in a water-miscible solvent.

In an 82^{nd} embodiment of the first aspect which is also an embodiment of the 81^{st} embodiment of the first aspect, the solvent is selected from the group comprising ethanol, acetone, 1-butanol, 2-butanol, tert.-butanol, 3-methyl-1-butanol, 2-methyl-1-propanol, 1-propanol, 2-propanol, dimethylsulfoxide, preferably from a group comprising ethanol, tert.-butanol and 1-butanol.

In an 83^{rd} embodiment of the first aspect which is also an embodiment of any one of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th} 31^{st}, 32^{nd} 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th}, 76^{th}, 77^{th}, 78^{th}, 79^{th}, 80^{th}, 81^{st} and 82^{nd} embodiment of the first aspect, the mean particle size of the lipid nanoparticles is from about 15 nm to about 400 nm.

In an 84^{th} embodiment of the first aspect which is also an embodiment of the 83^{rd} embodiment of the first aspect, the mean particle size of the lipid nanoparticles is from about 25 nm to about 200 nm.

In an 85^{th} embodiment of the first aspect which is also an embodiment of any one of the 83^{rd} and the 84^{th} embodiment of the first aspect, the mean particle size of the lipid nanoparticles is from about 40 nm to about 100 nm.

In an 86^{th} embodiment of the first aspect which is also an embodiment of any one of the 83^{rd}, 84^{th} and 85^{th} embodiment of the first aspect, the mean particle size of the lipid nanoparticles is determined by means of dynamic light scattering (DLS).

In an 87^{th} embodiment of the first aspect which is also an embodiment of each and any one of the 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th}, 76^{th}, 77^{th}, 78^{th}, 79^{th}, 80^{th}, 81^{st}, 82^{nd}, 83^{rd}, 84^{th}, 85^{th} and 86^{th} embodiment of the first aspect, the lipid nanoparticles have or show a Zeta-potential, wherein the Zeta-potential is from about -20 mV to about + 20 mV

In an 88^{th} embodiment of the first aspect which is also an embodiment of the 87^{th} embodiment of the first aspect, the Zeta-potential is from about - 10 mV to about + 10 mV.

In an 89^{th} embodiment of the first aspect which is also an embodiment of each and any one of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th}, 76^{th}, 77^{th}, 78^{th}, 79^{th}, 80^{th}, 81^{st}, 82^{nd}, 83^{rd}, 84^{th}, 85^{th}, 86^{th}, 87^{th} and 88^{th} embodiment of the first aspect, the payload molecule is selected from the group comprising a nucleic acid molecule or a polypeptide molecule comprising between 50 to 1000 amino acids and comprising an isoelectric point (pI) of >6.

In a 90^{th} embodiment of the first aspect which is also an embodiment of the 89^{th} embodiment of the first aspect, the payload molecule is a nucleic acid molecule.

In a 91^{st} embodiment of the first aspect which is also an embodiment of each and any one of the 89^{th} and 90^{th} embodiment of the first aspect, the nucleic acid is selected from the group comprising a ribonucleic acid molecule, a deoxyribonucleic acid molecule or a combination thereof.

In a 92^{nd} embodiment of the first aspect which is also an embodiment of each and any one of the 89^{th}, 90^{th} and 91^{st} embodiment of the first aspect, wherein the nucleic acid molecule is a single-stranded nucleic acid molecule, a double-stranded nucleic acid molecule or a partially double-stranded nucleic acid molecule.

In a 93^{rd} embodiment of the first aspect which is also an embodiment of each and any one of the 89^{th}, 90^{th}, 91^{st} and 92^{nd} embodiment of the first aspect, the nucleic acid comprises from about 15 to about 20000 nucleotides, preferably the nucleic acid molecule comprises from about 21 to about 10000 nucleotides, more preferably the nucleic acid molecule comprises from about 100 to about 8000 nucleotides and most preferably the nucleic acid molecule comprises from about 200 to about 7000 nucleotides.

In a 94^{th} embodiment of the first aspect which is also an embodiment of each and any one of the 89^{th}, 90^{th}, 91^{st}, 92^{nd} and 93^{rd} embodiment of the first aspect, the nucleic acid molecule is a functional nucleic acid molecule.

In a 95^{th} embodiment of the first aspect which is also an embodiment of the 94^{th} embodiment of the first aspect, the functional nucleic acid molecule is selected from the group comprising an mRNA, a Cas (CRISPR associated endonuclease protein)-encoding mRNA, a plasmid-DNA, an ssDNA (single stranded DNA), an Aptamer, a Spiegelmer, an siRNA molecule, an antisense molecule, an miRNA (micro RNA) molecule, an sgRNA (single guide RNA) molecule, an saRNA (self amplifying RNA) molecule, and a combination thereof.

In a 96^{th} embodiment of the first aspect which is also an embodiment of the 89^{th}. 90^{th}, 91^{st}, 92^{nd}, 93^{rd}, 94^{th} and 95^{th} embodiment of the first aspect, the lipid to nucleic acid mass ratio in the preparation is from 5 to 40, preferably from 10 to 35, even more preferably the lipid to nucleic acid mass ratio in the preparation is from 15 to 30, and most preferably the lipid to nucleic acid mass ratio in the preparation is about 28

In a 97^{th} embodiment of the first aspect which is also an embodiment of the 89^{th}. 90^{th}, 91^{st}, 92^{nd}, 93^{rd}, 94^{th}, 95^{th} and 96^{th} embodiment of the first aspect, the nucleic acid molecule is contained within the lipid nanoparticles.

In a 98^{th} embodiment of the first aspect which is also an embodiment of the 89^{th}. 90^{th}, 91^{st}, 92^{nd}, 93^{rd}, 94^{th}, 95^{th}, 96^{th} and 97^{th} embodiment of the first aspect, the lipid to nucleic acid mass ratio in the lipid preparation is from 5 to 40, preferably from 10 to 35, even more preferably the lipid to nucleic acid mass ratio in the lipid preparation is from 15 to 30, and most preferably the lipid to nucleic acid mass ratio in the lipid preparation is about 28.

In a 99^{th} embodiment of the first aspect which is also an embodiment of the 89^{th}. 90^{th}, 91^{st}, 92^{nd}, 93^{rd}, 94^{th}, 95^{th}, 96^{th}, 97^{th} and 98^{th} embodiment of the first aspect, the lipid to nucleic acid mass ratio in the lipid nanoparticles is from 5 to 40, preferably from 10 to 35, even more preferably the lipid to nucleic acid mass ratio in the lipid nanoparticles is from 15 to 30, and most preferably the lipid to nucleic acid mass ratio in the lipid nanoparticles is about 28

In a 100^{th} embodiment of the first aspect which is also an embodiment of the 89^{th} embodiment of the first aspect, the payload molecule is a polypeptide molecule comprising between 10 to 1000 amino acid residues, preferably between 50 to 900 amino acid residues and more preferably between 80 and 800 amino acid residues.

In a 101^{st} embodiment of the first aspect which is also an embodiment of the 89^{th} and 100st of the first aspect, the polypeptide comprises or has an isoelectric point (pI) of >6.

In a 102^{nd} embodiment of the first aspect which is also an embodiment of each and any one of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th}, 76^{th}, 77^{th}, 78^{th}, 79^{th}, 80^{th}, 81^{st}, 82^{nd}, 83^{rd}, 84^{th}, 85^{th}, 86^{th}, 87^{th}, 88^{th}, 89^{th}. 90^{th}, 91^{st}, 92^{nd}, 93^{rd}, 94^{th}, 95^{th}, 96^{th}, 97^{th}, 98^{th}, 99^{th}, 100^{th} and 101^{st} embodiment of the first aspect, the payload molecule is a therapeutically active agent or a precursor thereof.

In a 103^{rd} embodiment of the first aspect which is also an embodiment of each and any one of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, 40^{th}, 41^{st}, 42^{nd}, 43^{rd}, 44^{th}, 45^{th}, 46^{th}, 47^{th}, 48^{th}, 49^{th}, 50^{th}, 51^{st}, 52^{nd}, 53^{rd}, 54^{th}, 55^{th}, 56^{th}, 57^{th}, 58^{th}, 59^{th}, 60^{th}, 61^{st}, 62^{nd}, 63^{rd}, 64^{th}, 65^{th}, 66^{th}, 67^{th}, 68^{th}, 69^{th}, 70^{th}, 71^{st}, 72^{nd}, 73^{rd}, 74^{th}, 75^{th}, 76^{th}, 77^{th}, 78^{th}, 79^{th}, 80^{th}, 81^{st}, 82^{nd}, 83^{rd}, 84^{th}, 85^{th}, 86^{th}, 87^{th}, 88^{th}, 89^{th}. 90^{th}, 91^{st}, 92^{nd}, 93^{rd}, 94^{th}, 95^{th}, 96^{th}, 97^{th}, 98^{th}, 99^{th}, 100^{th}, 101^{st} and 102^{nd} embodiment of the first aspect, the preparation is suitable for local administration.

In a 104^{th} embodiment of the first aspect which is also an embodiment of the 103^{rd} embodiment of the first aspect, the local administration is intramuscular administration.

In a 105^{th} embodiment of the first aspect which is also an embodiment of the 103^{rd} embodiment of the first aspect, the local administration is subcutaneous administration.

In a 106^{th} embodiment of the first aspect which is also an embodiment of the 103^{rd} embodiment of the first aspect, the local administration is intradermal administration,

The problem underlying the present invention is solved in a second aspect, which is also a first embodiment of the second aspect, by the preparation according to the first aspect, including any embodiment thereof, for use in a method for the treatment and/or prevention of a disease in a subject, wherein the method comprises local administration of a payload molecule to the subject, preferably the method comprises local administration of the preparation.

In a second embodiment of the second aspect which is also an embodiment of the first embodiment of the second aspect, the local administration is intramuscular administration.

In a third embodiment of the second aspect which is also an embodiment of the first embodiment of the second aspect, the local administration is subcutaneous administration.

In a fourth embodiment of the second aspect which is also an embodiment of the first embodiment of the second aspect, the local administration is intravitreal administration.

In a fifth embodiment of the second aspect which is also an embodiment of the first embodiment of the second aspect, the local administration is intrathecal administration.

In a sixth embodiment of the second aspect which is also an embodiment of the first embodiment of the second aspect, the local administration is intratumoral administration.

In a seventh embodiment of the second aspect which is also an embodiment of the first embodiment of the second aspect, the local administration is intracerebral administration.

In an eighth embodiment of the second aspect which is also an embodiment of the first embodiment of the second aspect, the local administration is intradermal administration.

In a ninth embodiment of the second aspect which is also an embodiment of the first, second, third, fourth, fifth, sixth, seventh and eighth embodiment of the second aspect, the disease is a disease which can be treated and/or prevented by the payload molecule.

The problem underlying the present invention is solved in a third aspect by a pharmaceutical composition comprising a preparation as defined in the first aspect of the present invention, including any embodiment thereof, and a pharmaceutically acceptable excipient.

The problem underlying the present invention is solved in a fourth aspect, which is also a first embodiment of the fourth aspect, by the pharmaceutical composition according to the third aspect, including any embodiment thereof, for use in a method for the treatment and/or prevention of a disease in a subject, wherein the method comprises local administration of a payload molecule to the subject, preferably the method comprises local administration of the pharmaceutical composition.

In a second embodiment of the fourth aspect which is also an embodiment of the first embodiment of the fourth aspect, the local administration is intramuscular administration.

In a third embodiment of the fourth aspect which is also an embodiment of the first embodiment of the fourth aspect, the local administration is subcutaneous administration.

In a fourth embodiment of the fourth aspect which is also an embodiment of the first embodiment of the fourth aspect, the local administration is intravitreal administration.

In a fifth embodiment of the fourth aspect which is also an embodiment of the first embodiment of the fourth aspect, the local administration is intrathecal administration.

In a sixth embodiment of the fourth aspect which is also an embodiment of the first embodiment of the fourth aspect, the local administration is intratumoral administration.

In a seventh embodiment of the fourth aspect which is also an embodiment of the first embodiment of the fourth aspect, the local administration is intracerebral administration.

In an eighth embodiment of the fourth aspect which is also an embodiment of the first embodiment of the fourth aspect, the local administration is intradermal administration.

In a ninth embodiment of the fourth aspect which is also an embodiment of the first, second, third, fourth, fifth, sixth, seventh and eighth embodiment of the fourth aspect, the disease is a disease which can be treated and/or prevented by the payload molecule.

The problem underlying the present invention is solved in a 6^{th} aspect by the use of a first lipid in the preparing of a preparation for use according to the second aspect of the present invention, including any embodiment thereof, wherein the first lipid is a first lipid as defined in the first aspect of the present invention, including any embodiment thereof.

The problem underlying the present invention is solved in a 7^{th} aspect by the use of a second lipid in the preparing of a preparation for use according to the second aspect of the present invention, including any embodiment thereof, wherein the second lipid is a second lipid as defined in the first aspect of the present invention, including any embodiment thereof.

The problem underlying the present invention is solved in an 8^{th} aspect by the use of a third lipid in the preparing of a preparation for use according to the second aspect of the present invention, including any embodiment thereof, wherein the third lipid is a third lipid as defined in the first aspect of the present invention, including any embodiment thereof.

The problem underlying the present invention is solved in a 9^{th} aspect by the use of a fourth lipid in the preparing of a preparation for use according to the second aspect of the present invention, including any embodiment thereof, wherein the fourth lipid is a fourth lipid as defined in the first aspect of the present invention, including any embodiment thereof.

Without wishing to be bound by any theory, the present inventors surprisingly found that compared to strong cationic LNPs and LNPs comprising ionizable cationic lipids used in the prior art for intramuscular, subcutaneous, and intradermal administration of RNA, a lipid composition used in the preparation according to the present invention and, respectively, and LNPs formed by such lipid composition are superior to those strong cationic LNPs and the pH-sensitive cationic LNPs comprising ionizable cationic lipids. The LNPs formed by the lipid composition used in the preparation according to the present invention are also referred to herein as the LNPs of the present invention.

The mixture of lipids of the present invention is preferably strongly positively charged at lower pH values since the cationic lipids are positively charged at acidic pH values, but the anionic lipids are not charged anymore due to the protonation of their carboxylic groups. These strongly positively charged lipid systems are capable to interact electrostatically with RNAs, to complex RNAs and to form LNPs accordingly. Once the LNPs of the present invention are formed and the pH of the suspension medium is adjusted to a neutral pH value, the cationic lipids at the surface of the LNPs are still positively charged, but at the same time the negatively charges of the anionic lipids are regenerated due to the deprotonation of their carboxylic groups whereas the RNAs are still trapped inside the LNP particle. The molar ratios between cationic lipids and anionic lipids in these amphoteric LNP systems are preferably chosen in a way that at neutral pH values the number of cationic charges which are presented by the cationic lipids outweighs the number of the anionic charges which are presented by the anionic lipids. As a result, the LNP surface is decorated with almost the same number of positive and negative charges. Therefore, the resulting LNP surface can be described as overall neutral and in contrast to LNPs comprising ionizable cationic lipids of the prior art, it is not hydrophobic but hydrophilic.

In an embodiment of each and any aspect of the present invention including any embodiment thereof and as preferably used herein, a payload molecule is a compound which is active at a target. The activity is preferably an activity selected from the group comprising a biological activity, a chemical activity, a biochemical activity, a physiological activity, a pharmacological activity and a physical activity. As preferably used herein, a biological activity is an activity which causes a biological effect at the target. Preferably such biological effect is an increase or decrease in activity of a protein, a polypeptide or a peptide, wherein the protein or polypeptide is an enzyme, a structural protein, a scaffold protein, transmembrane protein, a channel protein, a secreted protein, a hormone, a mitochondrial protein, a receptor, a ligand, an epitope/ neoepitope/ antigen peptide (qualifying for MHC-I binding as 9-mer, or MHC-II binding as 13-25-mer) (see, e.g., Andreatta M et al., Bioinformatics 2016; 32:511-7), or a nucleic acids binding protein. Alternatively, such biological effect is an increase or decrease in activity or half-life of a nucleic acid molecule, or a localization, positioning, processing, transport, or packaging of a nucleic acid molecule. Such nucleic acid may be a deoxyribonucleic acid or a ribonucleic acid (see, e.g. Jurtz V et al., J Immunol 2017; 199:3360-8).

In an embodiment of each and any aspect of the present invention including any embodiment thereof and as preferably used herein, a target is selected from the group comprising a molecule, a subcellular structure, a cell, a tissue and an organ. Preferably, a target is targeted by the preparation of the present invention. Target function is envisioned to result in a gain-of-function phenotype, if replacement or substitution or supplementation of defective, aberrant, lost or low protein expression in a given cell by target is desired. Target function is also envisioned to result in a loss-of-function phenotype, if inhibition or abrogation of hyperactive, dominant-active, aberrant, or high protein expression in a given cell by target is desired. For example, in muscle cells genetic mutations are intended to be repaired on the DNA level, defective mutant protein variants are replaced (transient forced expression of active protein) or repaired (mRNA exon skipping). Transient forced protein expression is also intended to be secreted, endocytosed and presented extracellularly by any type of antigen-presenting cells, present or attracted at the site of administration.

In an embodiment of each and any aspect of the present invention including any embodiment thereof and as preferably used herein, local administration is an administration selected from the group comprising of intramuscular administration, subcutaneous administration, intravitreal administration, intrathecal administration, intratumoral administration, intracerebral administration, intramyocardial administration, intracoronary administration and intradermal administration

In an embodiment of each and any aspect of the present invention including any embodiment thereof and as preferably used herein, a lipid which is pH-independently positively charged is a lipid having an overall positive charge irrespective of the pH to which such lipid is exposed, preferably the pH to which such lipid is exposed in any pH of 0 to 14. More preferably, a lipid which is pH-independently positively charged is selected from the group comprising DOTAP (N-[1-(2,3-dioleoyloxy)propyl]-N,N,N-trimethylammonium chloride) and DOTMA (1,2-di-O-octadecenyl-3-trimethylammonium propane chloride).

In an alternative embodiment of each and any aspect of the present invention including any embodiment thereof and as preferably used herein, the overall pKa value of the LNP is measured by using the TNS assay (6-(p-Toluidino)-2-naphthalenesulfonic acid sodium salt assay). In a first step, a 4x TNS master buffer is prepared consisting of 100 mM citrate, 80 mM sodium phosphate, 80 mM ammonium acetate and 600 mM sodium chloride and a stock solution of 1 mg/ml TNS in a mixture of dimethylformamide and water (2/8 v/v). To make a series of 2x TNS Assay Buffer with different pH values (e.g., ranging from pH 3 to pH 10) for each pH value 20 ml of the 4x TNS master buffer is put into a tube such as a falcon tube, the pH is adjusted with HCl or NaOH to the target pH value and an appropriate amount of water is added to give 40 ml of a 2x TNS Assay Buffer. Subsequently, a working solution of the LNP the pKa of which is to be determined is prepared by adding 8 µl of TNS stock solution (dimethylformamide and water (2/8 v/v)) and 8 µl of a formulation (10 mM TRIS pH 7.5) containing said LNP (lipid concentration 2-8 mg/ml) to 2 ml water. The measurement is done in a black 96-well plate. For such purpose, 100 µl 2x TNS Assay Buffer per pH value is put in each well and 100 µl of the working solution is added to each well to give a final volume of 200 µl per well. The thus obtained final mixtures are incubated for 30 min at 37 °C and afterwards centrifuged for 1 min at 1000 rpm. The fluorescence intensity of the supernatant is read on a plate reader such as an Tecan M1000 plate reader at an excitation of 322 nm and an emission of 431 nm. After the measurement, the background fluorescence value of an empty well on the 96 well plate is subtracted from each probe /lipid /buffer mixture. The fluorescence intensity values are then normalized to the value at lowest pH. The normalized fluorescence intensity is subsequently plotted against pH and a line of best fit is provided. The point on the line of best fit at which the normalized fluorescence intensity is equal to 0.5 is determined and and the corresponding pH value is considered the pKa of the LNP.

In an embodiment of each and any aspect of the present invention including any embodiment thereof and as preferably used herein, an amphoteric overall neutrally charged lipid nanoparticle is a lipid nanoparticle where the sum of negative and positive charges born by the lipid nanoparticle is zero. Preferably the sum is zero if the lipid nanoparticle is exposed to a pH value of 7 to 8, more preferably the lipid particle is exposed to a pH value of 7.4. Consequently, the Zeta-potential of said lipid nanoparticle is zero ±5 mV.

In an embodiment of each and any aspect of the present invention including any embodiment thereof and as preferably used herein, a straight and/or branched chain C1-C4 hydrocarbon group means each and individually any of methyl, ethyl, n-propyl, isopropyl, n-butyl, isobutyl, *sec*-butyl and *tert*-butyl.

In an embodiment of each and any aspect of the present invention including any embodiment thereof and as preferably used herein, a neutral lipid is a lipid bearing either no charged molecular moiety or is a zwitterionic lipid molecule comprising the same amount of positively and negatively charged molecular moieties. Preferably the neutral lipid is a sterol or a phosphatidylethanolamine or a phosphatidylcholine.

In an embodiment of each and any aspect of the present invention including any embodiment thereof and as preferably used herein, a PEGlyated lipid is a compound comprising a lipid moiety and a PEG moiety attached to the lipid moiety. Preferably, the PEG moiety is covalently attached to the lipid moiety. The lipid moiety is preferably a sterol or a phosphatidylethanolamine or a diacylglycerol and more preferably the lipid moiety is selected from a group comprising 1,2-distearoyl-sn-glycero-3-phosphatidylethanolamine, 1,2-dipalmitoyl-sn-glycero-3-phosphatidylethanolamine, 1,2-dimyristoyl-sn-glycero-3-phosphatidylethanolamine, 1,2-distearoyl-rac-glycerol, 1,2-diplamitoyl-rac-glycerol and 1,2-dimyristoyl-rac-glycerol.

In an embodiment of each and any aspect of the present invention including any embodiment thereof and as preferably used herein, an outer surface of a lipid nanoparticle is a surface of a lipid nanoparticle which is facing any medium surrounding the lipid nanoparticle. Preferably, an outer surface of a lipid nanoparticle is opposite to an inner structure of a lipid nanoparticle.

In an embodiment of each and any aspect of the present invention including any embodiment thereof and as preferably used herein, a neutral pH value is a pH value of about 6.7 to about 7.7, preferably of about 6.9 to about 7.5, more preferably of about 7.2 to about 7.5.

In an embodiment of each and any aspect of the present invention including any embodiment thereof a negative charge of the lipid composition is provided by the first lipid.

In an embodiment of each and any aspect of the present invention including any embodiment thereof a positive charge of the lipid composition is provided by the second lipid.

In an embodiment of each and any aspect of the present invention including any embodiment thereof, the carboxylic group of the first lipid is present in a protonated or a deprotonated form. If present in the deprotonated form, the carboxylic group confers a negative charge to the first lipid.

In an embodiment of each and any aspect of the present invention including any embodiment thereof, the pKa value of the first lipid is determined by the carboxylic group group of the first lipid. Preferably, the pKa value of the first lipid is determined by the pKa value of the carboxylic group. More preferably, the pKa value of the carboxylic group is the pKa value of the first lipid.

In an embodiment of each and any aspect of the present invention including any embodiment thereof, the functional group of the second lipid is present in a protonated or nonprotonated form. If present in the protonated form, the functional group confers a positive charge to the second lipid.

In an embodiment of each and any aspect of the present invention including any embodiment thereof, the second lipid is (a) a cationic lipid having at least one molecular moiety with a pKa value between greater 9.5 and 13.5, or (b) a cationic lipid which is pH-independently positively charged.

In an embodiment of each and any aspect of the present invention including any embodiment thereof and as preferably used herein, the term alkyl group of 12 to 20 hydrocarbons comprises an alkyl group of 12 hydrocarbons, an alkyl group of 13 hydrocarbons, an alkyl group of 14 hydrocarbons, an alkyl group of 15 hydrocarbons, an alkyl group of 16 hydrocarbons, an alkyl group of 17 hydrocarbons, an alkyl group of 18 hydrocarbons, an alkyl group of 19 hydrocarbons and an alkyl group of 20 hydrocarbons.

In an embodiment of each and any aspect of the present invention including any embodiment thereof and as preferably used herein, the term branched alkyl group is an alkyl group having a backbone of hydrocarbon atoms, wherein at least one further hydrocarbon is covalently attached to a carbon atom of the hydrocarbons of the backbone at a position different from the hydrocarbon at either end of the backbone.

In an embodiment of each and any aspect of the present invention including any embodiment thereof and as preferably used herein, the term unsaturated alkyl group is an alkyl group comprising at least two hydrocarbons and at least one double bond covalently linking two hydrocarbons. It will be appreciated by a person skilled in the art that an unsaturated alkyl group comprises one or more double bonds. In a preferred embodiment, the unsaturated alkyl group comprises one, two, three or four double bonds. It will also be appreciated by a person skilled in the art that the double bond can be located at between any two adjacent hydrocarbons of the alkyl group. Preferably, in case the unsaturated alkyl group comprises two or more double bonds, such double bonds are arranged as double bonds separated by two single bonds. It will also be appreciated by a person skilled in the art that the double bond is either a cis double bond or a trans double bond. Preferably the double bond is a cis configurated double bond.

In an embodiment of each and any aspect of the present invention including any embodiment thereof, compound CHEMS (Cholesterol hemisuccinate) is of the following formula:

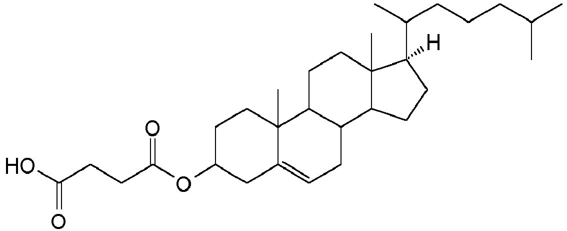

Preferably the stereocenters are defined as shown in the following formula:

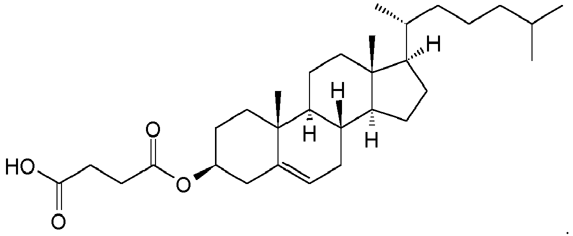

In an embodiment of each and any aspect of the present invention including any embodiment thereof, compound Alkyl carboxylic acids is of the following formula: 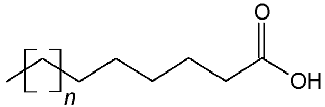 wherein n is = 1, 2, 3, 4, 5, 6, 7, 8, 9, 10 or 11.

In an embodiment of each and any aspect of the present invention including any embodiment thereof, compound Diacyl glycerol hemisuccinates is of the following formula: 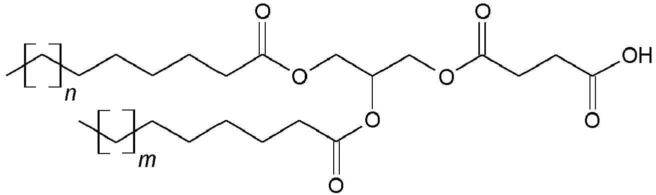 wherein n is 1, 2, 3, 4, 5, 6, 7, 8, 9, 10 or 11, and m is 1, 2, 3, 4, 5, 6, 7, 8, 9, 10 or 11.

In an embodiment of each and any aspect of the present invention including any embodiment thereof, compound Phosphatidylserines is of the following formula:

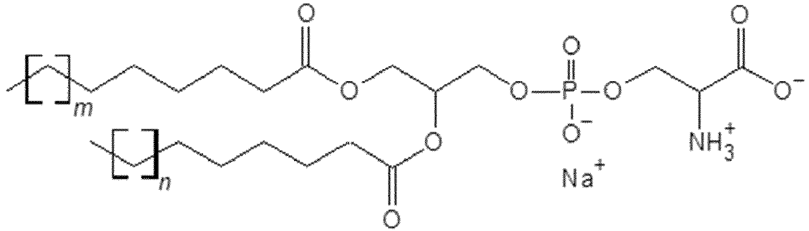

Preferably the stereocenters are defined as shown in the following formula: 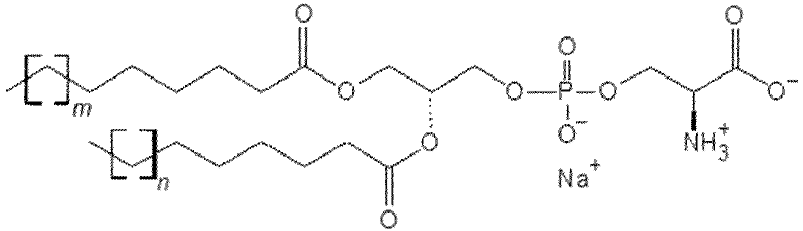 wherein n is 1, 2, 3, 4, 5, 6, 7, 8, 9, 10 or 11, and m is 1, 2, 3, 4, 5, 6, 7, 8, 9, 10 or 11.

In an embodiment of each and any aspect of the present invention including an embodiment thereof, β-(L-arginyl)-L-2,3-diamino propionic acid-N-palmityl-N-oleyl-amide) is of the following formula:

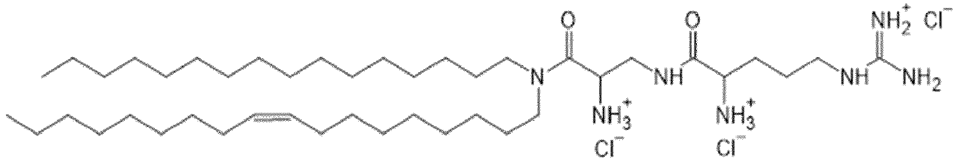

Preferably the stereocenters are defined as shown in the following formula:

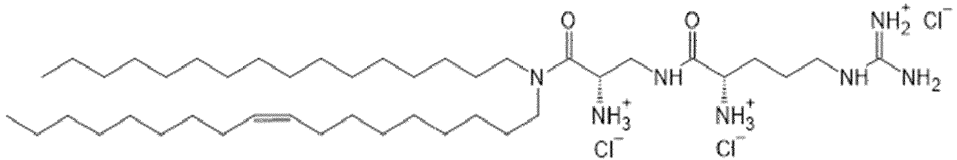

In an embodiment of each and any aspect of the present invention including an embodiment thereof, compound L-arginyl-β-alanine-N-palmityl-N-oleyl-amide is of the following formula:

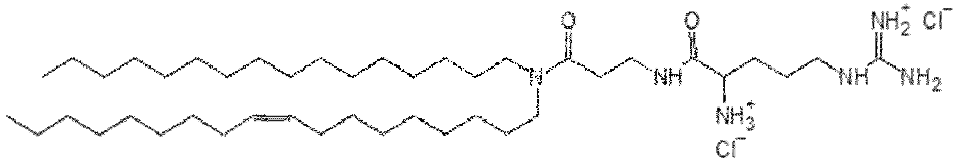

Preferably the stereocenters are defined as shown in the following formula:

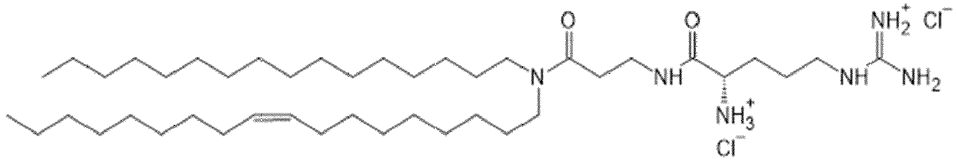

In an embodiment of each and any aspect of the present invention including an embodiment thereof, compound DOTAP (N-[1-(2,3-dioleoyloxy)propyl]-N,N,N-trimethylammonium methyl-chloride) is of the following formula:

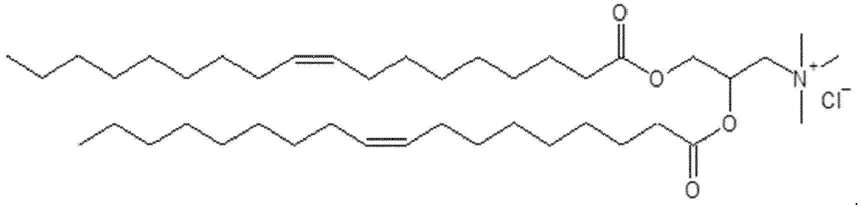

In an embodiment of each and any aspect of the present invention including an embodiment thereof, compound DOTMA (1,2-Di-O-octadecenyl-3-trimethylammonium propane (preferably as chloride salt)) is of the following formula:

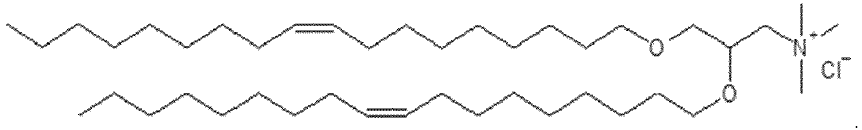

In an embodiment of each and any aspect of the present invention including an embodiment thereof, compound DC-cholesterol (3β-[N-(N',N'-dimethylaminoethane)-carbamoyl]cholesterol hydrochloride) is of the following formula:

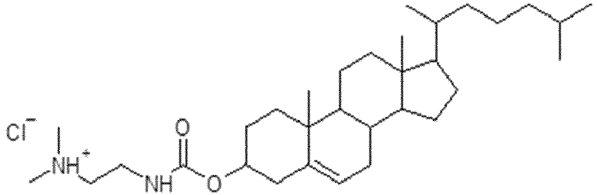

Preferably the stereocenters are defined as shown in the following formula:

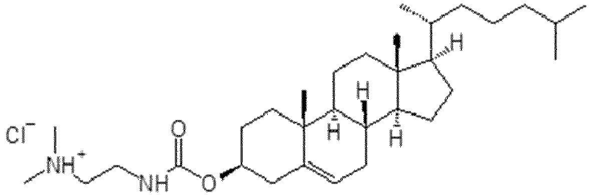

In an embodiment of each and any aspect of the present invention including an embodiment thereof and as preferably used herein a zoosterol is an animal derived sterol.

In an embodiment of each and any aspect of the present invention including an embodiment thereof and as preferably used herein, a phytosterol is a plant derived sterol.

In an embodiment of each and any aspect of the present invention including an embodiment thereof and as preferably used herein, a zwitterionic phospholipid is a phospholipid which comprises an equal number of positively and negatively charged functional groups.

In an embodiment of each and any aspect of the present invention including an embodiment thereof, compound DSPC (1,2-Distearoyl-sn-glycero-3-phosphoethanolcholine) is of the following formula:

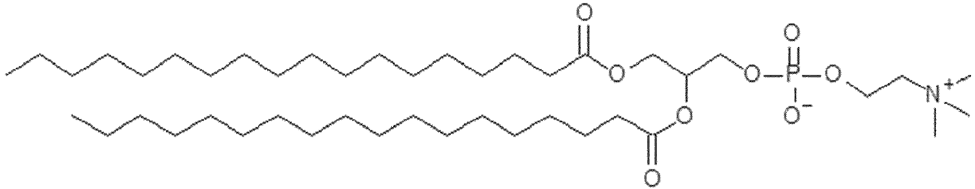

Preferably the stereocenters are defined as shown in the following formula:

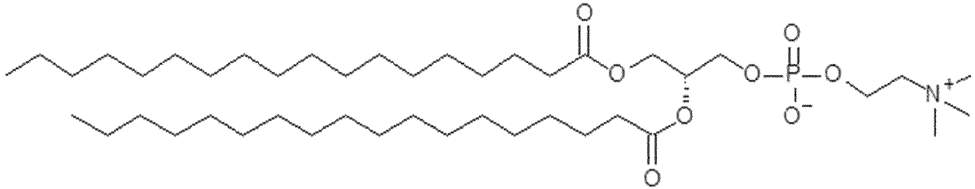

In an embodiment of each and any aspect of the present invention including an embodiment thereof, compound DMPC (1,2-Dimyristoyl-sn-glycero-3-phosphoethanolamine) is of the following formula:

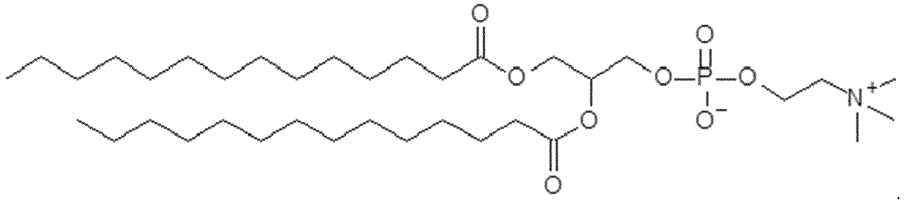

Preferably the stereocenters are defined as shown in the following formula:

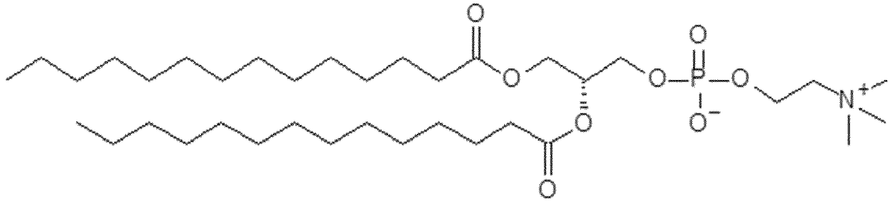

In an embodiment of each and any aspect of the present invention including an embodiment thereof, compound DSPE (1,2-Distearoyl-sn-glycero-3-phosphoethanolamine) is of the following formula:

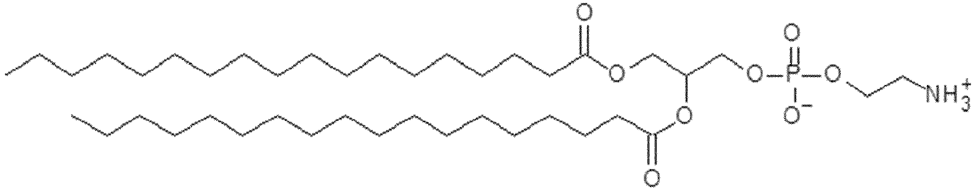

Preferably the stereocenters are defined as shown in the following formula:

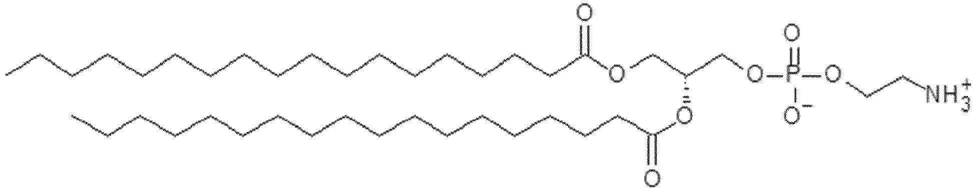

In an embodiment of each and any aspect of the present invention including an embodiment thereof, compound DMPE (1,2-Dimyristoyl-sn-glycero-3-phosphoethanolamine) is of the following formula:

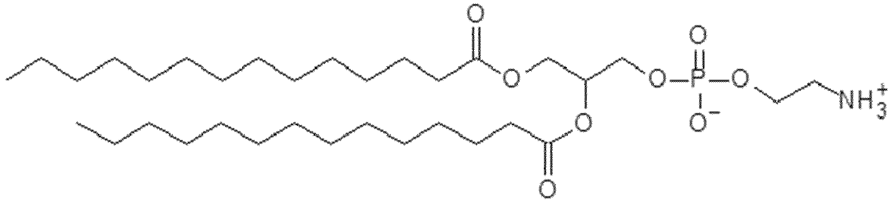

Preferably the stereocenters are defined as shown in the following formula:

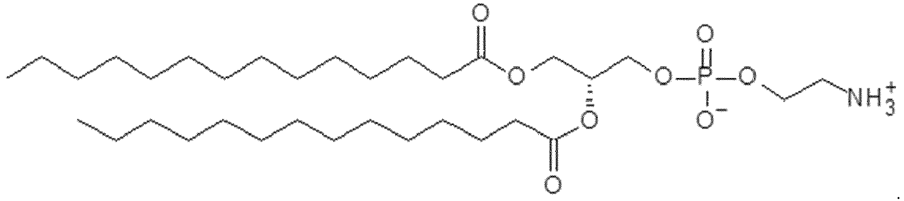

In an embodiment of each and any aspect of the present invention including an embodiment thereof, compound DLPE (1,2-Dilauroyl-sn-glycero-3-phosphoethanolamine) is of the following formula:

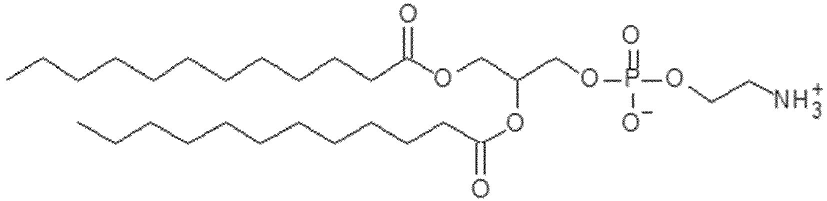

Preferably the stereocenters are defined as shown in the following formula:

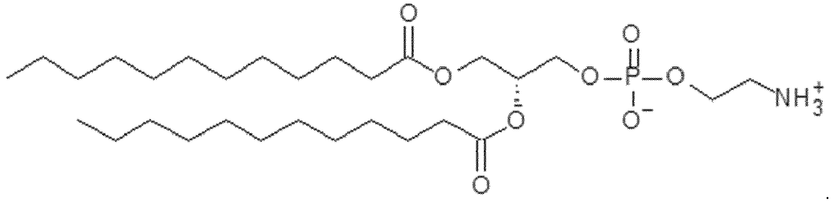

In an embodiment of each and any aspect of the present invention including an embodiment thereof, compound DPhyPE (1,2-(7R,11R) Diphytanoyl-sn-glycero-3-phosphoethanolamine) is of the following formula:

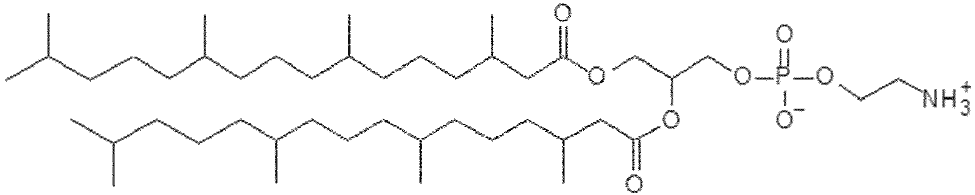

Preferably the stereocenters are defined as shown in the following formula:

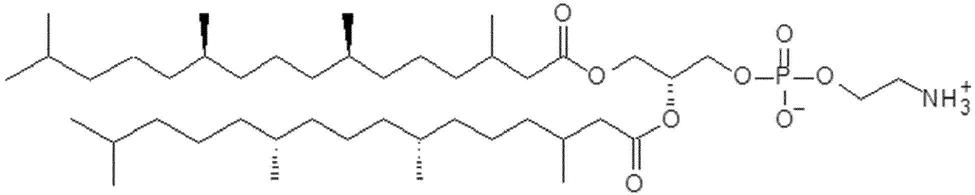

In an embodiment of each and any aspect of the present invention including an embodiment thereof, compound DOPE (1,2-Dioleoyl-sn-glycero-3-phosphoethanolamine) is of the following formula:

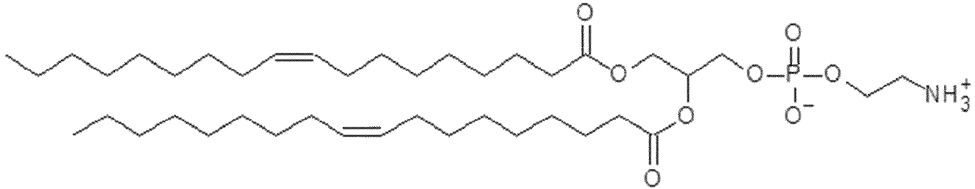

Preferably the stereocenters are defined as shown in the following formula:

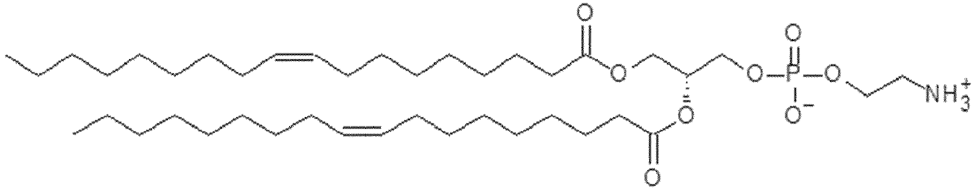

In an embodiment of each and any aspect of the present invention including an embodiment thereof, compound POPE ((1-Palmitoyl-2-oleoyl-sn-glycero-3-phosphoethanolamine) is of the following formula:

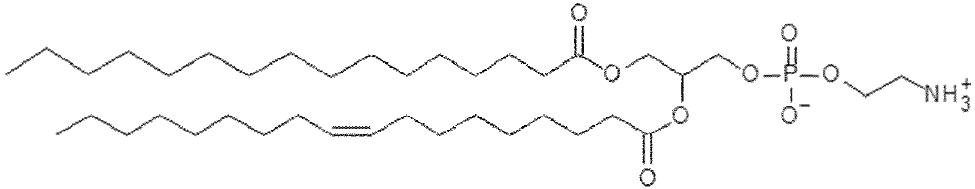

Preferably the stereocenters are defined as shown in the following formula:

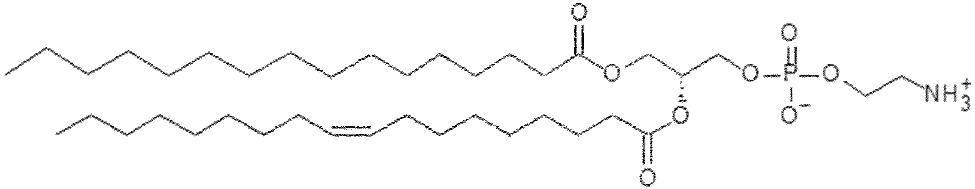

In an embodiment of each and any aspect of the present invention including an embodiment thereof, compound cholesterol is of the following formula:

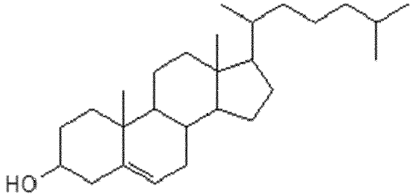

Preferably the stereocenters are defined as shown in the following formula:

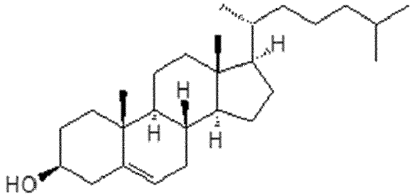

In an embodiment of each and any aspect of the present invention including an embodiment thereof, compound Stigmasterol (Stigmasta-5,22-dien-3-ol) is of the following formula:

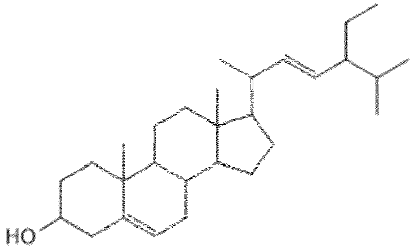

Preferably the stereocenters are defined as shown in the following formula:

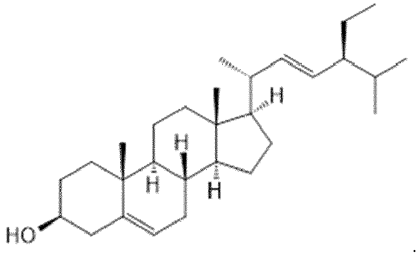

In an embodiment of each and any aspect of the present invention including an embodiment thereof and as preferably used herein, a shielding lipid is a lipid which provides steric stabilization of the lipid nanoparticles and also provides in vivo for a reduction of unintended interactions of the lipid nanoparticle surface with proteinaceous body fluids which may otherwise cause aggregation or decomposition of the LNPs. Consequently, it allows for a sustained *in vivo* delivery of the lipid nanoparticle payload to the target cells and thus for a more efficient delivery and prolonged functional bio-activity of the LNP payload. The term shielding also means that elements of the immune system or other defense or removal mechanisms of the body into which such lipid composition is administered, do not immediately interact with the lipid composition again increasing its functional bioavailability in a living organism. Insofar, the shielding lipid acts as an anti-opsonizing compound. Without wishing to be bound by any mechanism or theory, it seems that the shielding lipid forms a cover or coat which reduces the surface area of the lipid composition available for interaction with its environment which would otherwise result in the lipid composition to fuse with other lipids or being bound by factors of the human and animal body, respectively, at a time which is too early for such interaction although it has to be acknowledged that at a later stage, i. e. after a prolonged time upon administration of the lipid composition, such interaction is usually preferred or desired at least to a certain extent so as to provide the delivery of the payload of the lipid composition and preparation of the present invention, respectively.

The shielding compound is preferably a biologically inert molecular moiety covalently bound to a lipidic moiety. Depending on the hydrophobicity of the lipidic moiety, e.g. the alkyl chain length of the lipidic moiety ranging from C12 to C18 carbons, i.e. 12, 13, 14, 15, 16, 17, 18, 19 and 20 hydrocarbons, the shielding compound can be designed in a way, that it is able to dissociate from the lipid nanoparticle over time, rendering the nanoparticle surface more accessible for intended interactions with membranes of the target cells.

In an embodiment of each and any aspect of the present invention including an embodiment thereof, compound mPEG-500-DSPE (1,2-distearoyl-sn-glycero-3-phosphoethanolamine-N-[methoxy(polyethylene glycol)-500] is of the following formula:

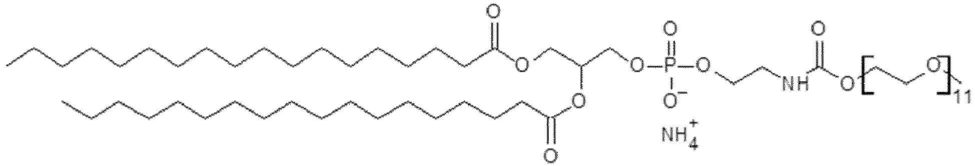

Preferably the stereocenters are defined as shown in the following formula:

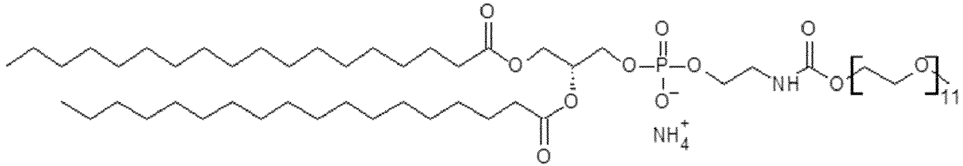

Preferably, compound mPEG-5oo-DSPE is present and used, respectively, as ammonium salt.

In an embodiment of each and any aspect of the present invention including an embodiment thereof, compound mPEG-1000-DSPE (1,2-distearoyl-sn-glycero-3-phosphoethanolamine-N-[methoxy(polyethylene glycol)-1000] is of the following formula:

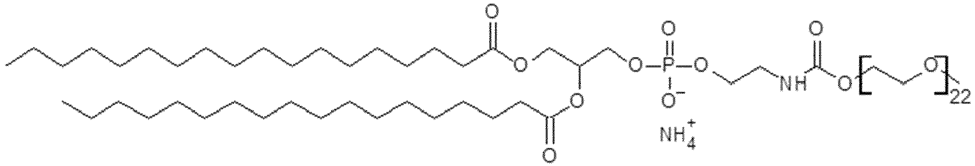

Preferably the stereocenters are defined as shown in the following formula:

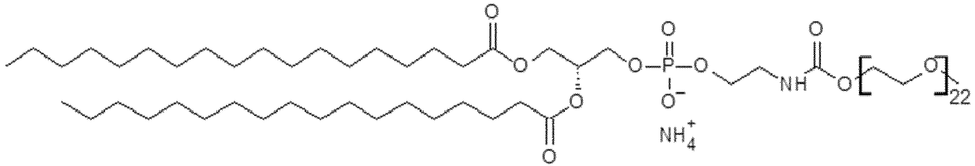

Preferably, compound mPEG-1000-DSPE is present and used, respectively, as ammonium salt.

In an embodiment of each and any aspect of the present invention including an embodiment thereof, compound mPEG-2000-DSPE (1,2-distearoyl-sn-glycero-3-phosphoethanolamine-N-[methoxy(polyethylene glycol)-2000] is of the following formula:

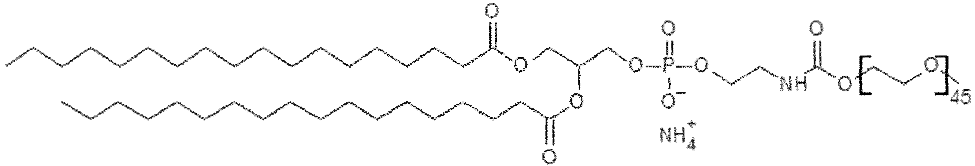

Preferably the stereocenters are defined as shown in the following formula:

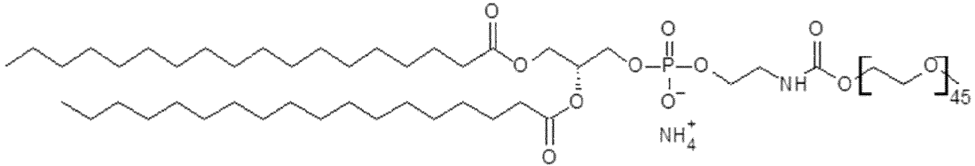

Preferably, compound mPEG-2000-DSPE is present and used, respectively, as ammonium salt.

In an embodiment of each and any aspect of the present invention including an embodiment thereof, compound mPEG-5000-DSPE (1,2-distearoyl-sn-glycero-3-phosphoethanolamine-N-[methoxy(polyethylene glycol)-5000] is of the following formula:

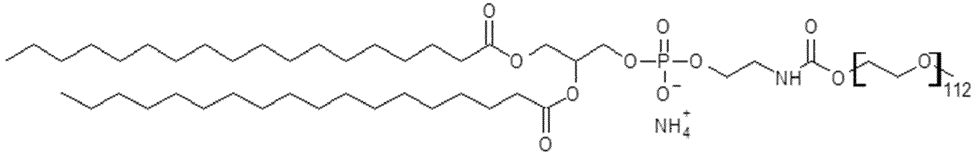

Preferably the stereocenters are defined as shown in the following formula:

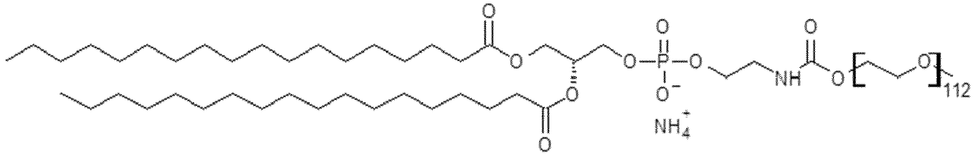

Preferably, compound mPEG-5000-DSPE is present and used, respectively, as ammonium salt.

In an embodiment of each and any aspect of the present invention including an embodiment thereof, compound mPEG-DSPE (1,2-distearoyl-sn-glycero-3-phosphoethanolamine-N-[methoxy(polyethylene glycol)] is of the following formula:

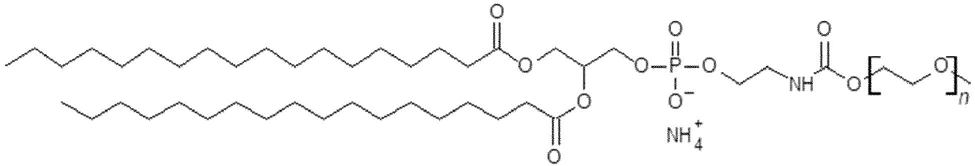

Preferably the stereocenters are defined as shown in the following formula:

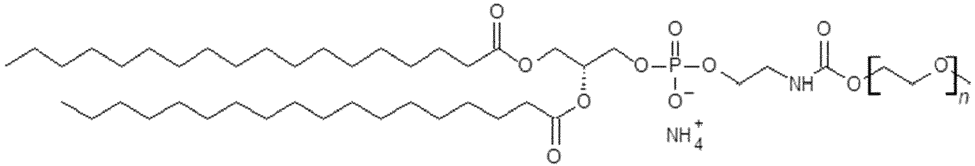

Preferably, compound mPEG-DSPE is present and used, respectively, as ammonium salt.

In an embodiment of each and any aspect of the present invention including an embodiment thereof, compound mPEG-2000-DMPE (1,2-dimyristoyl-sn-glycero-3-phosphoethanolamine-N-[methoxy(polyethylene glycol)-2000] is of the following formula:

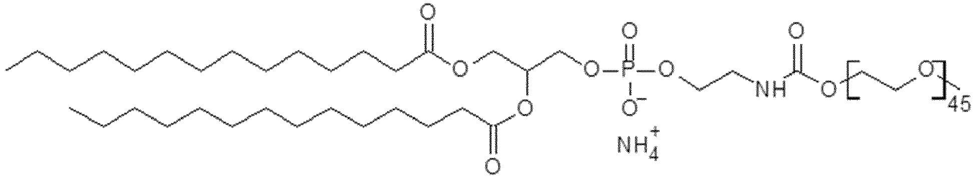

Preferably the stereocenters are defined as shown in the following formula:

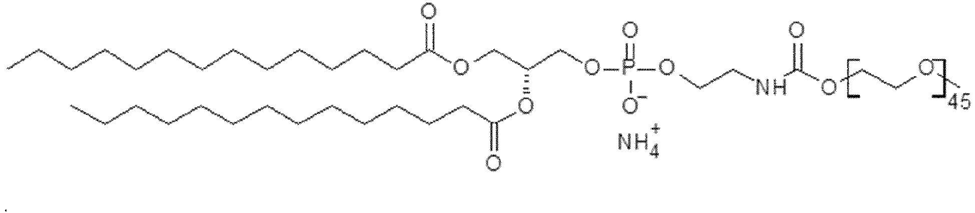

Preferably, compound mPEG-2000-DMPE is present and used, respectively, as ammonium salt.

In an embodiment of each and any aspect of the present invention including an embodiment thereof, compound mPEG-2000-DMG (1,2-dimyristoyl-rac-glycero-3-methoxypolyethylene glycol-2000) is of the following formula:

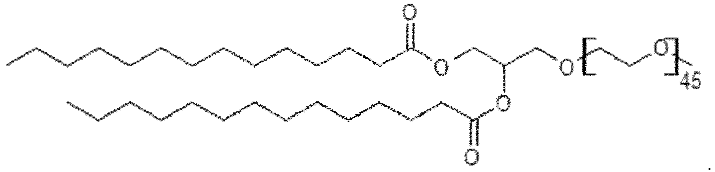

In an embodiment of each and any aspect of the present invention including an embodiment thereof, compound mPEG-2000-DPG (1,2-dipalmitoyl-rac-glycero-3-methoxypolyethylene glycol-2000) is of the following formula:

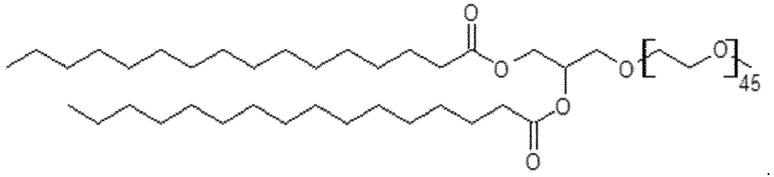

In an embodiment of each and any aspect of the present invention including an embodiment thereof, compound mPEG-2000-DSG (1,2-dipalmitoyl-rac-glycero-3-methoxypolyethylene glycol-2000) is of the following formula:

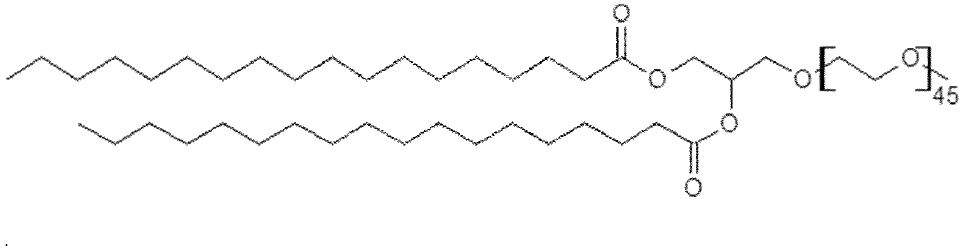

In an embodiment of each and any aspect of the present invention including an embodiment thereof, compound mPEG-2000-C-DMA (N-[(methoxy poly(ethylene glycol)2000)carbamyl]-1,2-dimyristyloxlpropyl-3-amine) is of the following formula:

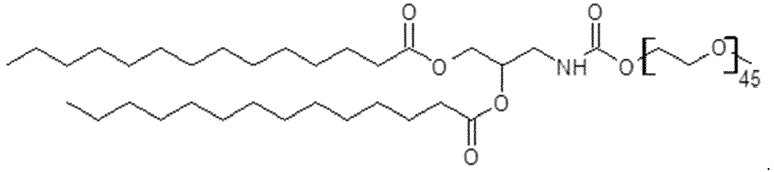

In an embodiment of each and any aspect of the present invention including an embodiment thereof, compound 2-(methoxyPEG-2000)-N,N-dioctadecylacetamide is of the following formula:

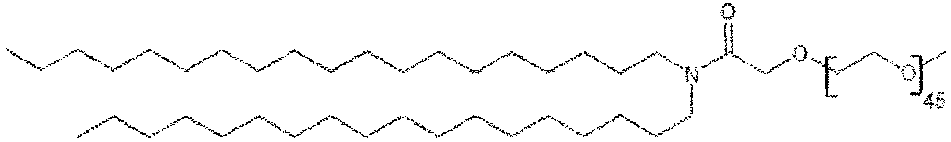

In an embodiment of each and any aspect of the present invention including an embodiment thereof, compound 2-(methoxyPEG-2000)-N,N-ditetradecylacetamide is of the following formula:

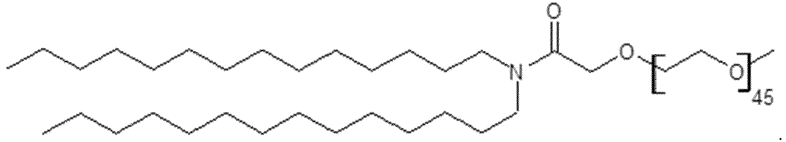

In an embodiment of each and any aspect of the present invention including an embodiment thereof, compound mPEG-2000-C8-Ceramide (N-octanoyl-sphingosine-1-{succinyl[methoxy(polyethylene glycol)2000]}) is of the following formula:

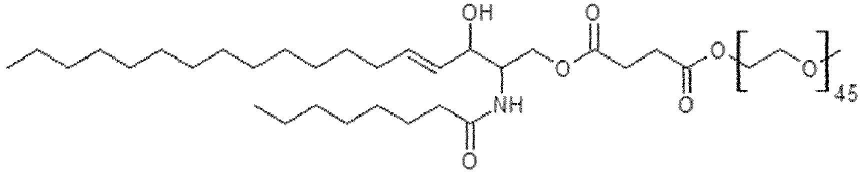

Preferably the stereocenters are defined as shown in the following formula:

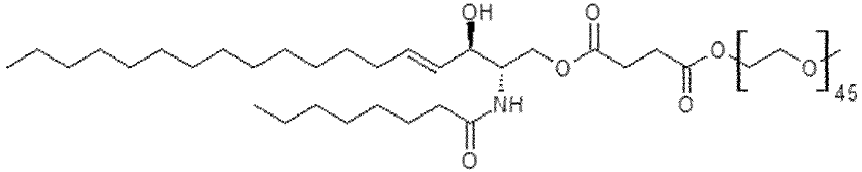

In an embodiment of each and any aspect of the present invention including any embodiment thereof, compound mPEG-2000-C16-Ceramide (N-palmitoyl-sphingosine-1-{succinyl[methoxy(polyethylene glycol)2000]}) is of the following formula:

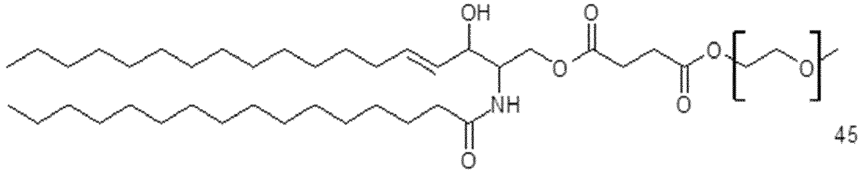

Preferably the stereocenters are defined as shown in the following formula:

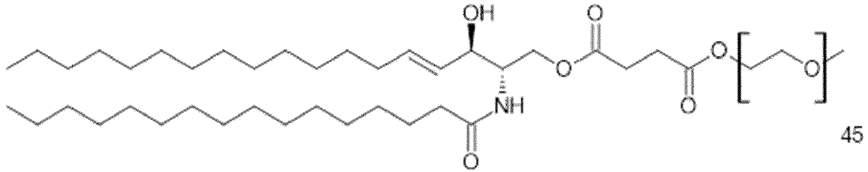

In an embodiment of each and any aspect of the present invention including any embodiment thereof, the mean particle size of the lipid nanoparticles is determined by means of dynamic light scattering (DLS) using a Zetasizer Ultra (Malvern Panalytical Ltd, Malvern, UK). All measurements are preferably carried out in an aqueous buffer as dispersant, which is a 270 mM sucrose solution buffered with 10 mM TRIS at pH 7.4. DLS is, for example, disclosed in more detail in Stetefeld, J., McKenna, S. A. & Patel, T. R. "Dynamic light scattering: a practical guide and applications in biomedical sciences". Biophys Rev 8, 409-427 (2016); and Thomas, J. C. "The determination of log normal particle size distributions by dynamic light scattering". Journal of Colloid and Interface Science 117, 187-192 (1987); the disclosure of which is herein incorporated by reference.

In an embodiment of each and any aspect of the present invention including any embodiment thereof, a zeta-potential such as a zeta-potential of lipid nanoparticles is measured by Laser Doppler Electrophoresis using a Zetasizer Ultra (Malvern Panalytical Ltd, Malvern, UK). All measurements are preferably carried out in an aqueous buffer as dispersant, which is a 270 mM sucrose solution buffered with 10 mM TRIS at pH 7.4. Laser Doppler Electrophoresis is, for example, described in more detail in Clogston, J. D. & Patri, A. K. in "Characterization of Nanoparticles Intended for Drug Delivery" 697, 63-70 (Humana Press, 2011); and Sze, A., Erickson, D., Ren, L. & Li, D. "Zeta-potential measurement using the Smoluchowski equation and the slope of the current-time relationship in electroosmotic flow", Journal of Colloid and Interface Science 261, 402-410 (2003), the disclosure of which is incorporated herein by reference.

In an embodiment of each and any aspect of the present invention including any embodiment thereof, the overall charge of the lipid nanoparticles is about ± 10 mV, preferably about ± 5 mV.

It will be appreciated by a person skilled in the art that the zeta potential of lipid nanoparticles is reflecting the overall charge of such lipid nanoparticles.

In an embodiment of each and any aspect of the present invention including any embodiment thereof, a nucleic acid molecule is a polymer of building blocks. Said building blocks are nucleotides. In an embodiment of each and any aspect of the present invention including any embodiment thereof, a ribonucleic acid molecule is a polymer of building blocks, wherein said building blocks are ribonucleotides. In an embodiment of each and any aspect of the present invention including any embodiment thereof, a deoxyribonucleic acid molecule is a polymer of building blocks, wherein said building blocks are deoxyribonucleotides, preferably 2' deoxyribonucleotides. In an embodiment of each and any aspect of the present invention including any embodiment thereof, said nucleotide building blocks are selected from a group comprising 2'-fluoro, 2'-methoxy modified ribonucleotides.

In an embodiment of each and any aspect of the present invention, including any embodiment thereof, a nucleic acid molecule is a D-nucleic acid molecule. In an embodiment and as preferably used herein, a D-nucleic acid molecule is a polymer of building blocks, wherein said building blocks are D-nucleotides. In a preferred embodiment of each and any aspect of the present invention including any embodiment thereof, a D-nucleic acid molecule is a D-ribonucleic acid molecule. In an embodiment and as preferably used herein, a D-ribonucleic acid molecule is a polymer of building blocks, wherein said building blocks are D-ribonucleotides. In an alternative preferred embodiment of each and any aspect of the present invention including any embodiment thereof, a D-nucleic acid molecule is a D-deoxyribonucleic acid molecule. In an embodiment and as preferably used herein, a D-deoxyribonucleic acid molecule is a polymer of building blocks, wherein said building blocks are D-deoxyribonucleotides.

In an embodiment of each and any aspect of the present invention including any embodiment thereof, a nucleic acid molecule is a D,L-nucleic acid molecule. In an embodiment and as preferably used herein, a D,L-nucleic acid molecule is a polymer of building blocks, wherein said building blocks are both D-nucleotides and L-nucleotides. It will be appreciated by a person skilled in the art that any relative ratio of D-nucleotides to L-nucleotides may be realized in such D,L-nucleic acid molecule. It will be further appreciated by a person skilled in the art that any arrangement of said D-nucleotides and L-nucleotides may be realized in such D,L-nucleic acid molecule. In a preferred embodiment of each and any aspect of the present invention including any embodiment thereof, a D,L-nucleic acid molecule is a D,L-ribonucleic acid molecule. In an embodiment and as preferably used herein, a D,L-ribonucleic acid molecule is a polymer of building blocks, wherein said building blocks are both D-ribonucleotides and L-ribonucleotides. In an alternative preferred embodiment of each and any aspect of the present invention including any embodiment thereof, a D,L-nucleic acid molecule is a D, L-deoxyribonucleic acid molecule. In an embodiment and as preferably used herein, a D,L-deoxyribonucleic acid molecule is a polymer of building blocks, wherein said building blocks are both D-deoxyribonucleotides and L-deoxyribonucleotides.

In an embodiment of each and any aspect of the present invention including any embodiment thereof and as preferably used herein, a single-stranded nucleic acid molecule is a nucleic acid molecule which consists of a single strand of nucleotides covalently attached to each other, preferably by means of a bond or linkage selected from the group consisting of a phosphodiester bond or a phosphothioether linkage. Preferably such linkage covalently links the OH group of a 3' C atom of a sugar moiety of a first nucleoside to a first OH group of a phosphate and the OH group of a 5' C atom of a sugar moiety of a second nucleoside to a second OH group of the phosphate.

In an embodiment of each and any aspect of the present invention including any embodiment thereof and as preferably used herein, a double-stranded nucleic acid molecule is a nucleic acid molecule which comprises a double-stranded structure. Said double-stranded structure is a structure where a first strand of nucleotides and a second strand of nucleotides are attached to each other. Such attachment is preferably selected from the group comprising a non-covalent attachment and a covalent attachment. In an embodiment, said non-covalent attachment is an attachment mediated or caused by one or more hydrogen bonds; preferably said non-covalent attachment is mediated or caused by Watson-Crick base pairing or by Hoogsteen base pairing between one or more of the nucleotides forming the first stand of nucleotides and the second strand of nucleotides. More preferably, all of the nucleotides of the first strand of nucleotides and all of the nucleotides of the second strand of nucleotides are involved in such base pairing, whereby one nucleotide of the first strand is base-pairing with one nucleotide of the second strand.

In an alternative embodiment, said covalent attachment is an attachment mediated by one or more covalent bonds between at least one nucleotide of the first strand of nucleotides forming the double-stranded structure and at least one nucleotide of the second strand of nucleotide forming the double-strand.

In an embodiment and as preferably used herein a functional nucleic acid molecule is a nucleic acid molecule having a function different from a structural nucleic acid molecule. In a preferred embodiment, a functional nucleic acid molecule is different from a ribosomal ribonucleic acid molecule, and different from a 7SL-RNA, and different from a ribozyme

In an embodiment and as preferably used herein, an mRNA is a nucleic acid molecule comprising 5'-> 3' direction, a Cap structure, a 5' untranslated region (5' UTR), a coding sequence typically starting with an AUG codon attached to a coding sequence (CDS) terminating with a stop codon, a 3' untranslated region (3' UTR) and a poly-A-tail. Preferably, the mRNA is made of ribonucleotides, more preferably of D-ribonucleotides.

In an embodiment and as preferably used herein, a Cas (CRISPR associated endonuclease protein)-encoding mRNA is an mRNA coding for CRISPR (Clustered Regularly Interspaced Short Palindromic Repeats) associated endonuclease protein. Cas proteins and derivatives thereof include for example Cas9 DNA nuclease (NCBI Reference Sequence (mRNA): NC_002737.2; NCBI Reference Sequence (Prot): NP_269215.1) or Cas13a-d RNA nuclease (e.g. LwaCas13a *Leptotrichia wadeii (*NCBI Reference Sequence: WP_021746003.1); PspCas13b *Prevotella sp. P5-125;* RfxCas13d *Ruminococcus flavefaciens* (NCBI Reference Sequence: WP_075424065.1))

In an embodiment and as preferably used herein, an aptamer is a single-stranded nucleic acid molecule that can form distinct and stable three-dimensional structures and specifically bind to a target molecule, wherein the aptamer is preferably made of D-nucleotides. Aptamers can be identified against several target molecules, e.g. small molecules, proteins, nucleic acids, and even cells, tissues and organisms and can inhibit the in vitro and/or in vivo function of the specific target molecule. Aptamers are usually identified by a target-directed selection process, called in vitro selection or Systematic Evolution of Ligands by Exponential Enrichment (abbr. SELEX) (Bock L.C. et al. (1992), Nature. 355(6360):564-6; Ellington & Szostak (1990) Nature, 346(6287):818-22; Tuerk C. and Gold L. (1990), Science. 249(4968):505-10). Non-modified aptamers are cleared rapidly from the bloodstream, with a half-life of minutes to hours, mainly due to nuclease degradation and clearance from the body by the kidneys, a result of the aptamer's inherently low molecular weight. Hence, in order to use aptamers therapeutically they have to be modified at the 2' position of the sugar. More preferably the binding of the aptamer to the target molecule is by means of non-covalent bonds, more preferably the by bonds different from Watson-Crick base pairing of Hoogsteen base pairing.

In an embodiment and as preferably used herein, a spiegelmer is a single-stranded nucleic acid molecule that can form distinct and stable three-dimensional structures and specifically bind to a target molecule, wherein the spiegelmer is preferably made of L-nucleotides. Spiegelmers can be identified against several target molecules, e.g., small molecules, proteins, nucleic acids, and even cells, tissues and organisms and can inhibit the in vitro and/or in vivo function of the specific target molecule. Spiegelmers are identified by a mirror-image selection published first in 1996 (Klussmann S. et al. (1996), Nat Biotechnol. 14(9):1112-5; Nolte A. et al. (1996), Nat Biotechnol. 14(9):1116-9). More preferably the binding of the aptamer to the target molecule is by means of non-covalent bonds, more preferably the by bonds different from Watson-Crick base pairing of Hoogsteen base pairing.

In an embodiment and as used herein, a plasmid DNA is a recombinant, circular double-stranded deoxyribonucleic acid molecule, comprising a nucleotide sequence, in particular an open reading frame under the control of a eukaryotic promoter sequence, encoding, e.g., a pharmacologically active polypeptide or protein. Alternatively, the eukaryotic promoter system is used to express a pharmacologically active nucleotide sequence encoding, a long non-coding RNA, a tRNA and/oran shRNA/ siRNA.

In an embodiment and as preferably used herein, an siRNA which is also referred to as short interfering RNA or silencing RNA in the art, is a class of double-stranded RNA non-coding RNA molecules, typically 20-24 base pairs in length and operating within the RNA interference (RNAi) pathway. It interferes with the expression of specific genes with complementary nucleotide sequences by degrading mRNA after transcription, preventing translation. siRNA molecules may be catalytically produced by the Dicer Enzyme from long double-stranded and small hairpin RNA molecules. The siRNA-induced post transcriptional gene silencing starts with the assembly of the RNA-induced silencing complex (RISC). The complex silences certain gene expression by cleaving the mRNA molecules coding the target genes. To begin the process, one of the two siRNA strands, the guide strand (anti-sense strand), will be loaded into the RISC while the other strand, the passenger strand (sense strand), is degraded. Certain Dicer enzymes may be responsible for loading the guide strand into RISC. Then, the siRNA scans for and directs RISC to - essentially - perfectly complementary sequence on the mRNA molecules. The cleavage of the mRNA molecules is thought to be catalyzed by the Piwi domain of Argonaute proteins of the RISC. The mRNA molecule is then cut precisely by cleaving the phosphodiester bond between the target nucleotides which are paired to siRNA residues 10 and 11, counting from the 5'end. This cleavage results in mRNA fragments that are further degraded by cellular exonucleases. The 5' fragment is degraded from its 3' end by exosome while the 3' fragment is degraded from its 5' end by 5' -3' exoribonuclease 1 (XRN1). Dissociation of the target mRNA strand from RISC after the cleavage allow more mRNA to be silenced. This dissociation process is likely to be promoted by extrinsic factors driven by ATP hydrolysis. Alternatively, siRNAs can be incorporated into an RNA-induced transcriptional silencing (RITS) complex. An active RITS complex will trigger the formation of heterochromatin around DNA matching the siRNA, effectively silencing the genes in that region of the DNA.

In an embodiment and as preferably used herein, an antisense molecule or antisense oligonucleotide is a single-stranded deoxyribonucleic acid or a single-stranded- ribonucleic acid.

In an embodiment and as preferably used herein, a natural or synthetic microRNA (miRNA, antagomir) is a small single-stranded non-coding RNA molecule containing about 22 nucleotides, that functions in gene silencing by post-transcriptional regulation of mRNA expression. miRNAs function via base-pairing with complementary sequences within mRNA molecules. As a result, these mRNA molecules are silenced, by one or more of the following processes: (1) cleavage of the mRNA strand into two pieces, (2) destabilization of the mRNA through shortening of its poly(A) tail and mRNA decay, and (3) by blocking mRNA translation.

In an embodiment and as preferably used herein, a single guide RNA (sgRNA) is a specific RNA sequence that recognizes the target DNA region of interest and directs a Cas nuclease there for editing. The sgRNA is made up of two parts: crispr RNA (crRNA), a nucleotide sequence of 17, 18, 19 or 20 nucleotides complementary to the target DNA, and a tracr RNA, which serves as a binding scaffold for the Cas nuclease. A sgRNA is a version of the naturally occurring two-piece guide RNA complex engineered into a single, continuous sequence. The simplified single-guide RNA is used to direct the Cas9 protein to bind and cleave a particular DNA sequence for genome editing.

In an embodiment and as preferably used herein, a self-amplifying RNA (saRNA) is encoding 5' and 3' CSE sequences, the Alphavirus nsP1-4 genes, a subgenomic promoter, and the protein coding region of a therapeutic protein or antigen. Following in situ translation, the nsP1-4 proteins form an RdRP complex which recognizes flanking CSE sequences and amplifies protein-encoding transcripts.

In an embodiment and as preferably used herein the lipid to nucleic acid mass ratio is the ratio of [the mass of the lipid composition of the preparation] to [the mass of the nucleic acid of the preparation]. More preferably, the mass of the nucleic acid of the preparation is the mass of the total of the nucleic acid molecules contained in the preparation.

In an embodiment and as preferably used herein, the nucleic acid molecule is contained within the lipid nanoparticles. It will be understood by a person skilled in the art that the term "contained within the lipid nanoparticles" preferably means that the majority of the individual nucleic acid molecules contained in the preparation is contained within the lipid nanoparticles. It is, however, also within the present invention that the individual nucleic acid molecules are, at least to a certain percentage, attached to the lipid nanoparticles. It is also within the present invention that the majority of the individual nucleic acid molecules are associated with the lipid composition of the preparation comprising a payload molecule and a lipid composition with the payload molecule and the lipid composition preferably being the one disclosed herein. It will be equally understood by a person skilled in the art that the disclosure specifically presented herein related to a nucleic acid molecule as a payload of the preparation equally applies to a polypeptide as a payload of the preparation.

In an embodiment and as preferably used herein, intramuscular administration is the injection of a substance into a muscle (e.g., striated muscle and smooth muscle, skeletal and cardiac muscle, sphincter muscles). It is one of several methods for parenteral administration of medications. Intramuscular injection may be preferred because muscles have larger and more numerous blood vessels than subcutaneous tissue, leading to faster absorption than subcutaneous or intradermal injections. Medication administered via intramuscular injection is not subject to the first-pass metabolism effect which affects oral medications.

Common sites for intramuscular injections include the deltoid muscle of the upper arm and the gluteal muscle of the buttock. In infants, the vastus lateralis muscle of the thigh is commonly used. Intramuscular administration after injection into skeletal muscle results in the delivery of larger volumes of a substance into the muscle tissue under the dermal and subcutaneous layer. The muscle is connected to the large network of blood and lymph vessels and offers depot effects for the substance and pronounced exposure to antigen presenting cells.

Subcutaneous administration refers to delivery into the subcutis region under the dermis and epidermis, whereas intradermal administration refers to delivery of the substance to the dermis directly. Both routes also offer depot effects to some extent. Intramyocardial administration refers to direct injections of substances into the cardiac muscle/ myocardium. Furthermore, non-skeletal muscles can be reached by direct injection of the substance into the given tissue, e.g., sphincter muscle of the urinary tract for stress urinary incontinence.

In an embodiment and as preferably used herein, the disease for the treatment and/or prevention of which the preparation of the invention may be used is a disease which can be treated and/or prevented by the payload molecule. Diseases include, but are not limited to: peripheral arterial disease (mRNA payload: VEGF165/COMP-Ang1, Follistatin-like 1), myositis (mRNA payload: anti-inflammatory cytokines), muscle hypertrophy (mRNA payload: Myostatin), diet-induced obesity (mRNA payload: IL-6, BDNF, FGF21), ischemia-reperfusion injury (mRNA payload: Myonectin), LAMA2-deficient muscular dystrophy (mRNA payload: αLNNd, Mini-agrin), depression (payload: kynurenine aminotransferase) Muscular and neuromuscular disorders comprise diseases such as myopathies, congenital myopathies, mitochondrial myopathies (payload tRNAs), muscular degeneration, muscular dystrophies, myositis, cancer (e.g. soft tissue sarcoma), atrophy (payload mRNA for various growth factors), hypertrophy, pseudohypertrophy, hypotonia, muscular spams, cachexia, glycogen storage diseases of muscle, muscle weakness (e.g. Myasthenia gravis) and cardiomyopathies and myocardial infarction.

In an embodiment and as preferably used herein, the prevention and/or treatment of a disease using a preparation of the underlying invention, comprises vaccination. In the context of the present invention, intramuscular delivery of a payload, such as a protein or an mRNA/ nucleic acid encoding a protein or polypeptide, acting as epitope or neoepitope (antigens) for antigen-presenting cells can be harnessed as vaccine to elicit an antigen specific immune response. Vaccination in the context of the present invention is intended to prevent or treat diseases such as infections, cancer, allergy and autoimmune diseases.

In an embodiment and as preferably used herein a pharmaceutically acceptable excipient is a pharmaceutically acceptable aqueous buffer system, preferably the buffer system has a physiological pH value and isoosmolar strength and further comprises a cryoprotectant, more preferably the buffer system has a pH value of 7.0 to 7.4 and an osmolality of 250 to 330 mosmol/kg and comprises a cryoprotectant selected from a group comprising sucrose and trehalose, and even more preferably the buffer system is a 10 mM phosphate or 10 mM TRIS/HCl buffer of pH 7.4 and comprises 270 mM Sucrose as cryoprotectant. Other pharmaceutically acceptable excipients are known to the person skilled in the art.

In an embodiment and as preferably used herein, a subject is a mammal selected from the group comprising human, monkey, rat and mouse, preferably a subject is a human being.

In an embodiment and as preferably used herein, a payload molecule is a therapeutically active agent.

In an embodiment and as preferably used herein, therapy is a method for the treatment and/or prevention of a disease in a subject, preferably the method comprises administering to the subject an agent which is suitable for or effective in the treatment and/or prevention of the disease.

The preparation of the present invention, particularly as disclosed in connection with the first aspect, relies on mixing a solution comprising the lipid components of the lipid composition with a solution comprising the mRNA, wherein the mixing is an in-line mixing. The mixing step is performed by the application of a microfluidic mixing device and can particularly either be done by the use of a staggered herringbone mixer device, a Dean Vortex bifurcating mixing device or a microfluidic hydrodynamic mixing device. These devices allow due to their special designed micro channels a rapid, non-turbulent and diffusion based mixing processes. A staggered herringbone mixer and its use in the preparation of particles as preferably contained in the composition of the invention, is, for example, described in Zhigaltsev, I. V. et al. "Bottom-Up Design and Synthesis of Limit Size Lipid Nanoparticle Systems with Aqueous and Triglyceride Cores Using Millisecond Microfluidic Mixing". Langmuir 28, 3633-3640 (2012), the disclosure of which is incorporated herein by reference. Microfluidic hydrodynamic mixing and its use in the preparation of particles as preferably contained in the composition of the invention, is, for example, described in Krzysztoń, R. et al. "Microfluidic self-assembly of folate-targeted monomolecular siRNA-lipid nanoparticles." Nanoscale 9, 7442-7453 (2017), the disclosure of which is incorporated herein by reference. Finally, Dean vortex bifurcating mixing and its use in the preparation of particles as preferably contained in the composition of the invention, is, for example, described in Chen, J. J., Chen, C. H. & Shie, S. R. "Optimal designs of staggered dean vortex micromixers." Int J Mol Sci 12, 3500-3524 (2011), the disclosure of which is incorporated herein by reference. In addition to the microfluidic mixing procedures, the mixing of a solution comprising the lipid components of the lipid composition with a solution comprising the mRNA can be performed using a confined impingement jet mixer [Knauer GmbH, Berlin, Germany].

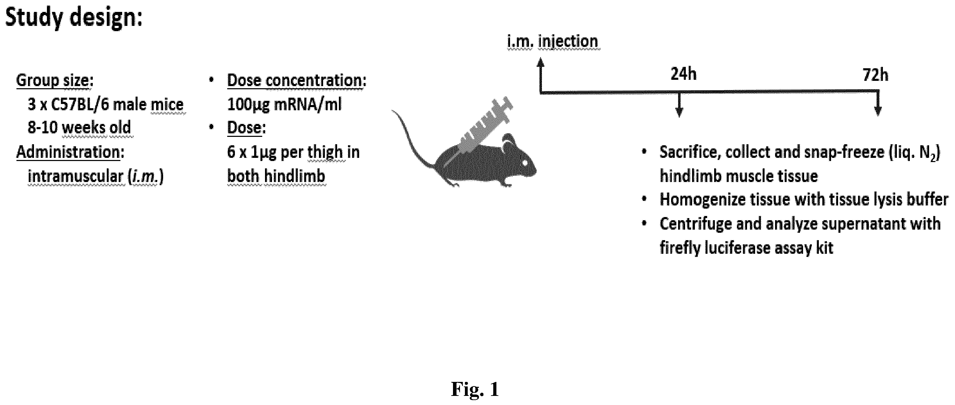

The present invention will be further illustrated by the following examples from which further features, embodiments and advantages may be taken, wherein
Fig. 1 shows the study design for in vivo assessment of luciferase expression after intramuscular injection into mice of FLuc-mRNA, FLuc-mRNA encapsulated into cationic LNP's, FLuc-mRNA encapsulated into overall neutral LNP's according to the present invention and FLuc-mRNA encapsulated into neutral LNP based on ionizable cationic lipids according to the prior art;

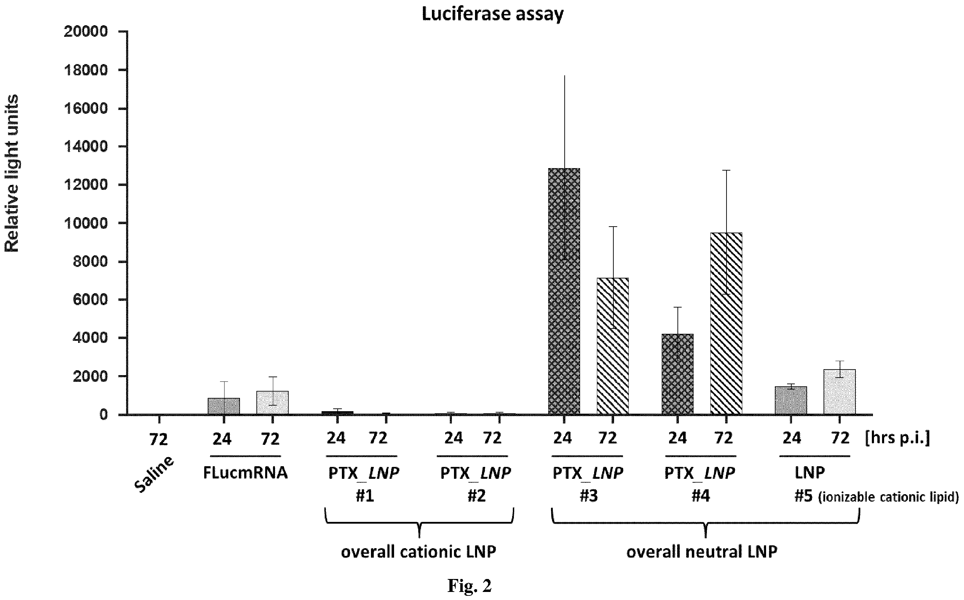

Fig. 2 is a diagram showing relative light units as indication of luciferase activity of various formulations, more specifically Fig. 2 shows the obtained luciferase expression results after intramuscular injection into mice of FLuc-mRNA, FLuc-mRNA encapsulated into cationic LNP's, FLuc-mRNA encapsulated into overall neutral LNP's according to the present invention and FLuc-mRNA encapsulated into neutral LNP based on ionizable cationic lipids according to the prior art;
Fig. 3 shows diagram I indicating a pH-dependent overall surface charge of lipid nanoparticles of an embodiment of the preparation according to the present invention, wherein the first lipid of the lipid composition is bearing a carboxylic group which is deprotonated in a pH-dependent manner, and the second lipid of the lipid composition is bearing three functional groups which are protonated in a pH-dependent manner;
Fig. 4 shows diagram II indicating a pH-dependent overall surface charge of lipid nanoparticles of an embodiment of the preparation according to the present invention, wherein the first lipid of the lipid composition is bearing a carboxylic group which is deprotonated in a pH-dependent manner, and the second lipid of the lipid composition is bearing two functional groups which are protonated in a pH-dependent manner; and
Fig. 5 shows diagram III indicating a pH-dependent overall surface charge of lipid nanoparticles of an embodiment of the preparation according to the present invention, wherein the first lipid of the lipid composition is bearing a carboxylic group which is deprotonated in a pH-dependent manner, and the second lipid of the lipid composition is bearing one functional group which is protonated in a pH-dependent manner.

### Example 1: Formulation of FLuc mRNA in lipid nanoparticles (PTX_LNP#1) for in vivo applications by intramuscular, subcutaneous or intradermal administration

For in vivo experiments, mRNA-LNPs were prepared in a formulation process with β-(L-Arginyl)-L-2,3-diamino propionic acid-N-palmityl-N-oleyl-amide as cationic lipid. Alternatively, other cationic lipids like L-Arginyl-β-alanine-N-palmityl-N-oleyl-amide can be used in an identical procedure to prepare similar mRNA-LNPs.

A lipid solution was prepared by dissolving the lipid components 2 β-(L-arginyl)-L-2,3-diamino propionic acid-N-palmityl-N-oleyl-amide : DPhyPE : mPEG-2000-DSPE at a molar ratio of 50:49:1 in ethanol. Subsequently, this lipid solution was mixed with a solution of CleanCap^{®} FLuc mRNA (5moU (=5-methoxy-uridine modification); Trilink Biotechnologies, San Diego) in water at a volume ratio (RNA solution : lipid solution) of 2:1 using a microfluidic mixer (NanoAssemblr^{®}; Precision Nanosystems, Vancouver, BC), applying an overall flow rate of 18 ml/min. The applied lipid and mRNA concentrations were adjusted in a way, that the total lipid to mRNA mass ratio of the thus obtained mRNA formulation equals 28. After the mixing process, the formulations were dialyzed against 10 mM TRIS buffered 270 mM Sucrose solution using 3.5 kDa MWCO Slide-A-Lyzer Dialysis Cassettes (Thermo Fisher Scientific). The formulation in the floating dialysis cassette was dialyzed for 2 hours at room temperature while gently stirring the dialysis buffer; the dialysis buffer was changed and dialyzed for another 2 hours at room temperature. Once again, the dialysis buffer was changed and dialyzed at 4 °C overnight. During the dialysis procedure, a total dialysis buffer of at least 300 times the volume of the sample was used. Instead of Sucrose, other sugars like Trehalose or Glucose can equally be used within the formulation process.

Subsequently, the thus obtained formulation was tested for particle size (Zetasizer Ultra instrument (Malvern Instruments Ltd, Malvern, UK), RNA encapsulation (Quant-iT RiboGreen RNA Assay Kit following manufacturer's (Thermo Fisher Scientific) protocol, and endotoxin content. Particle sizes were found to be between 60 to 90 nm (Z-Average) having a Zeta-potential of 35 mV +/- 10 mV. The formulation displayed greater than 90 % mRNA encapsulation. At this point, the final mRNA-concentration of the formulation was adjusted to 100 µg/ml. The obtained mRNA-LNP formulations were stored at -80 °C until further in vitro or in vivo use.

### Example 2: Formulation of FLuc mRNA in lipid nanoparticles (PTX_LNP#2) for in vivo applications by intramuscular, subcutaneous or intradermal administration

For in vivo experiments, mRNA-LNPs were prepared in a formulation process with β-(L-Arginyl)-L-2,3-diamino propionic acid-N-palmityl-N-oleyl-amide as cationic lipid. Alternatively, other cationic lipids like L-Arginyl-β-alanine-N-palmityl-N-oleyl-amide can be used in an identical procedure to prepare similar mRNA-LNPs.

A lipid solution was prepared by dissolving the lipid components 2 β-(L-arginyl)-L-2,3-diamino propionic acid-N-palmityl-N-oleyl-amide : Cholesterol : mPEG-2000-DSPE at a molar ratio of 70:29:1 in ethanol. Subsequently, this lipid solution was mixed with a solution of CleanCap^{®} FLuc mRNA (5moU; Trilink Biotechnologies, San Diego) in water at a volume ratio (RNA solution : lipid solution) of 3:1 using a microfluidic mixer (NanoAssemblr^{®}; Precision Nanosystems, Vancouver, BC), applying an overall flow rate of 18 ml/min. The applied lipid and mRNA concentrations were adjusted in a way, that the total lipid to mRNA mass ratio of the thus obtained mRNA formulation equals 14. After the mixing process, the formulations were dialyzed against 10 mM TRIS buffered 270 mM Sucrose solution using 3.5 kDa MWCO Slide-A-Lyzer Dialysis Cassettes (Thermo Fisher Scientific). The formulation in the floating dialysis cassette was dialyzed for 2 hours at room temperature while gently stirring the dialysis buffer; the dialysis buffer was changed and dialyzed for another 2 hours at room temperature. Once again, the dialysis buffer was changed and dialyzed at 4 °C overnight. During the dialysis procedure, a total dialysis buffer of at least 300 times the volume of the sample was used. Instead of Sucrose, other sugars like Trehalose or Glucose can equally be used within the formulation process.

Subsequently, the thus obtained formulation was tested for particle size (Zetasizer Ultra instrument (Malvern Instruments Ltd, Malvern, UK), RNA encapsulation (Quant-iT RiboGreen RNA Assay Kit following manufacturer's (Thermo Fisher Scientific) protocol, and endotoxin content. Particle sizes were found to be between 60 to 90 nm (Z-Average) having a Zeta-potential of 35 mV +/- 10 mV. The formulation displayed greater than 90 % mRNA encapsulation. At this point, the final mRNA-concentration of the formulation was adjusted to 100 µg/ml. The obtained mRNA-LNP formulations were stored at -80 °C until further in vitro or in vivo use.

### Example 3: Example 3: Formulation of FLuc mRNA in neutral lipid nanoparticles (PTX_LNP#3) for in vivo applications by intramuscular, subcutaneous or intradermal administration

For in vivo experiments, mRNA-LNPs were prepared in a formulation process with β-(L-Arginyl)-L-2,3-diamino propionic acid-N-palmityl-N-oleyl-amide as cationic lipid. Alternatively, other cationic lipids like L-Arginyl-β-alanine-N-palmityl-N-oleyl-amide can be used in an identical procedure to prepare similar mRNA-LNPs.

A lipid solution was prepared by dissolving the lipid components 2 β-(L-arginyl)-L-2,3-diamino propionic acid-N-palmityl-N-oleyl-amide : CHEMS : Cholesterol : mPEG-2000-DMPE at a molar ratio of 29:49:20:2 in ethanol. Subsequently, this lipid solution was mixed with a solution of CleanCap^{®} FLuc mRNA (5moU; Trilink Biotechnologies, San Diego) in acetate buffer (50 mM, pH 4) at a volume ratio (RNA solution : lipid solution) of 2:1 using a microfluidic mixer (NanoAssemblr^{®}; Precision Nanosystems, Vancouver, BC), applying an overall flow rate of 18 ml/min. The applied lipid and mRNA concentrations were adjusted in a way, that the total lipid to mRNA mass ratio of the thus obtained mRNA formulation equals 28. After the mixing process, the formulations were dialyzed against 10 mM TRIS buffered 270 mM Sucrose solution using 3.5 kDa MWCO Slide-A-Lyzer Dialysis Cassettes (Thermo Fisher Scientific). The formulation in the floating dialysis cassette was dialyzed for 2 hours at room temperature while gently stirring the dialysis buffer; the dialysis buffer was changed and dialyzed for another 2 hours at room temperature. Once again, the dialysis buffer was changed and dialyzed at 4 °C overnight. During the dialysis procedure, a total dialysis buffer of at least 300 times the volume of the sample was used. Instead of Sucrose, other sugars like Trehalose or Glucose can equally be used within the formulation process.

Subsequently, the thus obtained formulation was tested for particle size (Zetasizer Ultra instrument (Malvern Instruments Ltd, Malvern, UK), RNA encapsulation (Quant-iT RiboGreen RNA Assay Kit following manufacturer's (Thermo Fisher Scientific) protocol, and endotoxin content. Particle sizes were found to be between 60 to 90 nm (Z-Average) having a Zeta-potential of 0 mV ± 10 mV. The formulation displays greater than 90 % mRNA encapsulation. At this point, the final mRNA-concentration of the formulation was adjusted to 100 µg/ml. The obtained mRNA-LNP formulations were stored at -80 °C until further in vitro or in vivo use.

### Example 4: Example 4: Formulation of FLuc mRNA in lipid nanoparticles (PTX_LNP#4) for in vivo applications by intramuscular, subcutaneous or intradermal administration

For in vivo experiments, mRNA-LNPs were prepared in a formulation process with β-(L-Arginyl)-L-2,3-diamino propionic acid-N-palmityl-N-oleyl-amide as cationic lipid. Alternatively, other cationic lipids like L-Arginyl-β-alanine-N-palmityl-N-oleyl-amide can be used in an identical procedure to prepare similar mRNA-LNPs.

A lipid solution was prepared by dissolving the lipid components 2 β-(L-arginyl)-L-2,3-diamino propionic acid-N-palmityl-N-oleyl-amide : CHEMS : Cholesterol : mPEG-2000-DMPE at a molar ratio of 29:48:18:5 in ethanol. Subsequently, this lipid solution was mixed with a solution of CleanCap^{®} FLuc mRNA (5moU; Trilink Biotechnologies, San Diego) in acetate buffer (50 mM, pH 4) at a volume ratio (RNA solution : lipid solution) of 2:1 using a microfluidic mixer (NanoAssemblr^{®}; Precision Nanosystems, Vancouver, BC), applying an overall flow rate of 18 ml/min. The applied lipid and mRNA concentrations were adjusted in a way, that the total lipid to mRNA mass ratio of the thus obtained mRNA formulation equals 32. After the mixing process, the formulations were dialyzed against 10 mM TRIS buffered 270 mM Sucrose solution using 3.5 kDa MWCO Slide-A-Lyzer Dialysis Cassettes (Thermo Fisher Scientific). The formulation in the floating dialysis cassette was dialyzed for 2 hours at room temperature while gently stirring the dialysis buffer; the dialysis buffer was changed and dialyzed for another 2 hours at room temperature. Once again, the dialysis buffer was changed and dialyzed at 4 °C overnight. During the dialysis procedure, a total dialysis buffer of at least 300 times the volume of the sample was used. Instead of Sucrose, other sugars like Trehalose or Glucose can equally be used within the formulation process.

Subsequently, the thus obtained formulation was tested for particle size (Zetasizer Ultra instrument (Malvern Instruments Ltd, Malvern, UK), RNA encapsulation (Quant-iT RiboGreen RNA Assay Kit following manufacturer's (Thermo Fisher Scientific) protocol, and endotoxin content. Particle sizes were found to be between 40 to 90 nm (Z-Average) having a Zeta-potential of 0 mV ± 10 mV. The formulation displayed greater than 90 % mRNA encapsulation. At this point, the final mRNA-concentration of the formulation was adjusted to 100 µg/ml. The obtained mRNA-LNP formulations were stored at -80 °C until further in vitro or in vivo use.

### Example 5: In vivo assessment of Luciferase expression after intramuscular injection of different PTX LNPs into mice.

To assess the firefly luciferase (FLuc) expression after intramuscular injection of the various PTX_LNP's, a mouse study was designed as schematically depicted in Fig. 1. The group designation is shown in the following table, namely, group A was a saline group without any FLuc-mRNA serving as a vehicle control. Group B was the mere FLuc-mRNA without any lipids serving as a control, Group C was an overall positively charged FLuc-mRNA-LNP prepared as described in Example 1. Group D was an overall positively charged FLuc-mRNA-LNP prepared as described in Example 2. Group E was an overall neutral FLuc-mRNA-LNP according to the underlying invention which was prepared as described in Example 3 and which comprises 2 mol% mPEG2000-DMPE. Group F is an overall neutral FLuc-mRNA-LNP according to the underlying invention which was prepared as described in Example 4 and which comprises 5 mol% mPEG2000-DMPE. Group G was a neutral FLuc-mRNA-LNP based on an ionizable cationic lipid of the prior art. For said groups the luciferase expression was assessed 24 hours post injection, whereas for groups H to M, comprising the same samples as groups B to G, the luciferase expression was assessed 72 hours post injection. Each group consisted of three 8-10 weeks old C57BL/6 mice.

For treatment, the mice received per thigh (left & right hindlimb) 6×10µl of each test substance as outlined in the Table 1 below by intramuscular injection using Omnican 50U-100 syringes with 30G needles (0.30 mm × 12 mm). Therefore, prior to injection the thighs were shaved, and mice received 6 × 10µl injections per thigh muscle in a distance of approximately 2-3 mm each with the cannula applied 4-5 mm deep into the muscle tissue.

24 hours or 72 hours respectively, mice were sacrificed, and the total thigh muscle tissue was snap-frozen in liquid nitrogen. For luciferase assay, the frozen muscle tissue was pulverized in a dry-ice pre-cooled mortar and 60-90mg of the tissue was homogenized in PROMEGA Luciferase Cell Culture Lysis lysis buffer (10µl/mg tissue powder) for 5min/50Hz using a bead raptor (QIAGEN TissueLyser LT).

The homogenates were centrifuged and the expressed luciferase in the supernatants was measured using the PROMEGA Nano-Glo^{®} Dual-Luciferase^{®} Reporter Assay System kit. In brief, undiluted tissue lysate was mixed with an equal volume of luciferin containing assay buffer and luminescence was measured immediately using a TECAN infinite 200 Pro luminometer.

The results are presented in Fig. 2. In comparison to the overall cationic LNP's (PTX LNP#2 and PTX LNP#3) and the plain FLuc-mRNA, the overall charge-neutral LNP's (PTX LNP#3 and PTX LNP#4) displayed a significantly higher Luciferase expression after intramuscular injection. Furthermore, also compared to the overall charge-neutral LNP of the prior art (based on an ionizable cationic lipid), the overall charge-neutral LNP's according to the underlying invention (PTX_LNP#3 and PTX_LNP#4) showed a significantly higher Luciferase expression after intramuscular injection. Interestingly, for PTX_LNP#4 comprising a higher amount of mPEG-DMPE lipid (5% versus 2% in PTX_LNP#3), the Luciferase expression after 72 hours was significantly higher than after 24 hours, indicating a sustained biological activity of the PTX_LNP#4.

**Table 1: Group designation (dose/timepoints) of mouse experiment for Luciferase expression after intramuscular injection.**

| | **Group** | **Dose Group µg mRNA/thigh** | **Sacrifice hours p.t.** |
|---|---|---|---|
| **A** | Saline control | - | 24 h |
| **B** | FLuc-mRNA* | 6 µg (6 × 10 µl) | 24 h |
| **C** | PTX_LNP#1 | 6 µg (6 × 10 µl) | 24 h |
| **D** | PTX_LNP#2 | 6 µg (6 × 10 µl) | 24 h |
| **E** | PTX_LNP#3 | 6 µg (6 × 10 µl) | 24 h |
| **F** | PTX_LNP#4 | 6 µg (6 × 10 µl) | 24 h |
| **G** | LNP#5 | 6 µg (6 × 10 µl) | 24 h |
| **H** | FLuc-mRNA* | 6 µg (6 × 10 µl) | 72 h |
| **I** | PTX_LNP#1 | 6 µg (6 × 10 µl) | 72 h |
| **J** | PTX_LNP#2 | 6 µg (6 × 10 µl) | 72 h |
| **K** | PTX_LNP#3 | 6 µg (6 × 10 µl) | 72 h |
| **L** | PTX_LNP#4 | 6 µg (6 × 10 µl) | 72 h |
| **M** | LNP#5 | 6 µg (6 × 10 µl) | 72 h |

| | | | |
|---|---|---|---|
| *"naked" CleanCap^{®} FLuc mRNA (5moU) dissolved in 0.9% saline without any lipids at a conc. of 0.100 mg/ml | | | |

The features of the present invention disclosed in the specification, the claims and/or the drawings may both separately and in any combination thereof be material for realizing the invention in various forms thereof.

## Claims

1. A preparation for use in therapy,
wherein therapy comprises local administration of the preparation to a subject, wherein the local administration is selected from the group consisting of intramuscular administration, subcutaneous administration, intravitreal administration, intrathecal administration, intratumoral administration, intracerebral administration and intradermal administration,
wherein the preparation comprises lipid nanoparticles,
wherein the lipid nanoparticles comprise a lipid composition and a therapeutically active agent, and, at a pH of between 7 and 8, are amphoteric overall neutrally charged lipid nanoparticles,
wherein the lipid composition comprises a first lipid and a second lipid,
wherein the first lipid, at a pH of between 6.7 and 7.7, is an anionic lipid having at least one molecular moiety having a pKa value between 4 and 7,
wherein the second lipid is
(a) at a pH of between 6.7 and 7.7, a cationic lipid having at least one molecular moiety the pKa value of which is as follows: 9.5 < pKa ≤ 13.5, or
(b) a cationic lipid which is pH-independently positively charged; and
wherein the therapeutically active agent is a therapeutically active nucleic acid molecule.

2. The preparation for use of claim 1, wherein the lipid composition comprises a third lipid and/or a fourth lipid, wherein the third lipid is a neutral lipid, and the fourth lipid is a PEGylated lipid.

3. The preparation for use of any one of claims 1 to 2, wherein a surface of the lipid nanoparticles, preferably an outer surface of the lipid nanoparticles, provides about the same number of positive (cationic) charges and of negative (anionic) charges at a neutral pH value of between 6.7 and 7.7.

4. The preparation for use of any one of claims 1 to 3, wherein the molar ratio of the first lipid to the second lipid is such that the number of negative (anionic) charges of the lipid composition and the number of positive (cationic) charges of the lipid composition is about identical at a pH of between 6.7 and 7.7.

5. The preparation for use of any one of claims 1 to 4, wherein the first lipid is selected from the group consisting of CHEMS (Cholesterol hemisuccinate), an alkyl carboxylic acid, a diacyl glycerol hemisuccinate, and a diacyl-phosphatidylserine, wherein preferably the acylgroup is a saturated or unsaturated branched or straight chain alkyl-group of 12 to 20 hydrocarbons.

6. The preparation for use of any one of claims 1 to 5, wherein the second lipid is selected from the group consisting of β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium chloride), DOTMA (1,2-di-O-octadecenyl-3-trimethylammonium propane (chloride salt)), and DC-Chol (3β-[N-(N',N'-dimethylaminoethane)-carbamoyl]cholesterol hydrochloride), preferably the second lipid is β-(L-Arginyl)-L-2,3-diamino propionic acid-N-palmityl-N-oleyl-amide.

7. The preparation for use of any one of claims 2 to 6, wherein the third lipid is (a) a zwitterionic phospholipid selected from the group consisting of phosphatidyl ethanolamine, phosphatidyl choline or (b) an uncharged sterol lipid selected from the group comprising a zoosterol and a phytosterol.

8. The preparation for use of claim 7, wherein the zwitterionic phospholipid is selected from the group consisting of DSPC (1,2-Distearoyl-sn-glycero-3-phosphocholine), DMPC (1,2-dimyristoyl-sn-glycero-3-phosphocholine), DSPE (1,2-distearoyl-sn-glycero-3-phosphoethanolamine), DMPE (1,2-dimyristoyl-sn-glycero-3-phosphoethanolamine), DLPE (1,2-Lauroyl-sn-glycero-3-phosphoethanolamine), DPhyPE (1,2-Diphytanoyl-sn-glycero-3-phosphoethanolamine), DOPE (1,2-Dioleoyl-sn-glycero-3-phosphoethanolamine), and POPE (1-palmitoyl-2-oleoyl-sn-glycero-3-phosphoethanolamine).

9. The preparation for use of any one of claims 2 to 8, wherein the fourth lipid is selected from the group comprising methoxyPEG-DSPE, methoxyPEG-DMPE, methoxyPEG-DMG, methoxyPEG-DPG, methoxyPEG-DSG, methoxyPEG-c-DMA, 2(methoxyPEG)-N,N-dioctadecylacetamide, 2(methoxyPEG)-N,N-ditetradecylacetamide, methoxyPEG-C8-Ceramide, and methoxyPEG-C16-Ceramide.

10. The preparation for use of any one of claims 2 to 9, wherein the lipid composition comprises:
- CHEMS as the first lipid,
- β-(L-Arginyl)-L-2,3-diamino propionic acid-N-palmityl-N-oleyl-amide as the second lipid,
- cholesterol as the third lipid,
- methoxyPEG2000-DMPE as the fourth lipid

11. The preparation for use of any one of claims 2 to 10, wherein the molar ratio of the lipid composition is
(a):
- 20 - 80 mol% of the first lipid
- 20 - 80 mol% of the second lipid
- 10 - 50 mol% of the third lipid, and
- 1 - 10 mol% of the fourth lipid,
wherein the overall lipid content of the lipid composition is 100 mol%, or
(b):
- 20 - 60 mol% of the first lipid
- 20 - 60 mol% of the second lipid
- 10 - 40 mol% of the third lipid, and
- 1 - 5 mol% of the fourth lipid,
wherein the overall lipid content of the lipid composition is 100 mol%.

12. The preparation for use of claim 11, wherein
the lipid composition comprises
- 49 mol% of CHEMS as the first lipid,
- 29 mol% of β-(L-Arginyl)-L-2,3-diamino propionic acid-N-palmityl-N-oleyl-amide as the second lipid,
- 20 mol% of cholesterol as the third lipid and
- 2 mol% mPEG2000-DMPE as the fourth lipid;
or
the lipid composition comprises:
- 48 mol% of CHEMS as the first lipid
- 29 mol% of β-(L-Arginyl)-L-2,3-diamino propionic acid-N-palmityl-N-oleyl-amide as the second lipid
- 18 mol% of cholesterol as the third lipid and
- 5 mol% of methoxyPEG2000-DMPE as the fourth lipid.

13. The preparation for use of any one of claims 1 to 12, wherein the mean particle size of the lipid nanoparticles is from about 15 nm to about 400 nm, preferably from about 25 nm to about 200 nm and more preferably from about 40 nm to about 100 nm, as determined by means of dynamic light scattering (DLS).

14. The preparation for use of any one of claims 1 to 13, wherein a Zeta-potential of the lipid nanoparticles is from about -20 mV to about + 20 mV, preferably from about - 10 mV to about + 10 mV.

15. The preparation for use of any one of claims 1 to 14, wherein the therapeutically active nucleic acid molecule is selected from the group consisting of an mRNA, a Cas (CRISPR associated endonuclease protein)-encoding mRNA, a plasmid-DNA, a ssDNA (single stranded DNA), an Aptamer, a Spiegelmer, an siRNA molecule, an antisense molecule, an miRNA (micro RNA) molecule, an sgRNA (single guide RNA) molecule, an saRNA (self-amplifying RNA) molecule and a combination thereof.

16. The preparation for use of any one of claims 1 to 15, wherein therapy comprises a method for the treatment and/or prevention of a disease in a subject, and wherein the method comprises local administration of the preparation to the subject.
